# EUROPEAN PATENT SPECIFICATION

(11) **EP 3 261 679 B1**
(45) Date of publication and mention of the grant of the patent: **03.05.2023**
(21) Application number: 16706413.8
(22) Date of filing: 19.02.2016
(51) Int. Cl.: A61K 48/00

(54) **GENE THERAPY TO IMPROVE VISION**
GENTHERAPY ZUR VERBESSERUNG DER SEHKRAFT
THÉRAPIE GÉNIQUE POUR AMÉLIORER LA VISION

(30) Priority: 23.02.2015 GB 201503008
(43) Date of publication of application: 03.01.2018
(73) Proprietor: UCL Business Ltd, London WC1E 6BT (GB)
(72) Inventor: RIZZI, Matteo, London EC1V 9EL (GB); ALI, Robin, London EC1V 9EL (GB); SMITH, Alexander, London EC1V 9EL (GB); NISHIGUCHI, Koji, London EC1V 9EL (GB)
(74) Representative: J A Kemp LLP
(86) International application number: PCT/GB2016/050419
(87) International publication number: WO 2016/135457

(56) References cited:
- WO-A1-2009/127705
- WO-A1-2013/124477
- WO-A2-2012/167109
- ELSA LHÉRITEAU ET AL: "Successful Gene Therapy in the RPGRIP1-deficient Dog: a Large Model of Cone-Rod Dystrophy", MOLECULAR THERAPY, vol. 22, no. 2, 4 October 2013 (2013-10-04), pages 265-277, XP055266023, GB ISSN: 1525-0016, DOI: 10.1038/mt.2013.232
- KOJI M. NISHIGUCHI ET AL: "Gene therapy restores vision in rd1 mice after removal of a confounding mutation in Gpr179", NATURE COMMUNICATIONS, vol. 6, 23 January 2015 (2015-01-23), page 6006, XP055266591, DOI: 10.1038/ncomms7006
- XUE HAN ET AL: "A High-Light Sensitivity Optical Neural Silencer: Development and Application to Optogenetic Control of Non-Human Primate Cortex", FRONTIERS IN SYSTEMS NEUROSCIENCE, vol. 5, 13 April 2011 (2011-04-13), XP055265835, DOI: 10.3389/fnsys.2011.00018
- M. E. BURNS ET AL: "Lessons from Photoreceptors: Turning Off G-Protein Signaling in Living Cells", PHYSIOLOGY, vol. 25, no. 2, 1 April 2010 (2010-04-01), pages 72-84, XP055266715, US ISSN: 1548-9213, DOI: 10.1152/physiol.00001.2010 cited in the application
- JILLIAN N. PEARRING ET AL: "R9AP targeting to rod outer segments is independent of rhodopsin and is guided by the SNARE homology domain", MOLECULAR BIOLOGY OF THE CELL, vol. 25, no. 17, 1 September 2014 (2014-09-01), pages 2644-2649, XP055689422, US ISSN: 1059-1524, DOI: 10.1091/mbc.e14-02-0747
- I Sandoval ET AL: "AAV-Directed Overexpression of R9AP and RGS9-1 in Rat Retina", Investigative Ophthalmology & Visual Science, 1 May 2006 (2006-05-01), page Abstract 853, XP055689442, Retrieved from the Internet: URL:https://iovs.arvojournals.org/article. aspx?articleid=2390957 [retrieved on 2020-04-27]

## Description

The present invention relates to the use of gene therapy vectors to improve vision in patients.

### BACKGROUND OF THE INVENTION

In many mammalian species including mice and humans, the number of rod photoreceptors that mediate vision under dim light outnumbers greatly that of cone photoreceptors (Curcio et al, 2000). However, in an industrialised world where illumination allows cones to operate throughout the day, rod-mediated vision is less important. Many patients with absent rod function from birth are identified only incidentally and, in fact, cannot recognize their abnormal vision (Dryja, 2000). On the contrary, when cone dysfunction is present, patients are always symptomatic and often suffer visual handicap dependent on the degree of their cone dysfunction. In some conditions, however, only (or mostly) the cones are lost or dysfunctional and rods remain relatively preserved. For example, achromatopsia is a severe hereditary retinal dystrophy with a complete absence of cone function from birth but, presumably, with a normal rod function (Hess et al, 1986; Nishiguchi et al, 2005). Mutations in multiple genes including *CNGA3* (Kohl et al 1998); and *PDE6C* (Chang et al, 2009; Thiadens et al, 2009) have been associated with the disease. Each of the disease causing genes encodes an essential component of the cone phototransduction cascade that translates light into an electric signal by causing hyperpolarization of the photoreceptor cell. In age related macular degeneration (AMD), visual impairment is caused primarily by degeneration of the cone-rich fovea in the central macula. Thus patients lose central vision and acuity, but often have relatively well preserved peripheral macula and thus have some useful residual vision that is limited by the paucity of cones outside the fovea.

Rods are highly sensitive to light, which enables them to perceive a small amount of light in dim conditions. Cones, on the contrary, are less sensitive, but are capable of processing large amounts of light and continuously convey visual signals in daylight. This functional difference is, in part, due to the efficiency of the deactivation machinery of photo-signalling, the GTPase complex composed of RGS9, R9AP (also known as RGS9BP), and Gβ5. RGS9 is the catalytic component that hydrolyses the GTP coupled to the G-protein, whereas R9AP and Gβ5 are the essential constitutive subunits (Burns et al, 2009; Burns et al, 2010). Importantly, R9AP tethers the complex to the disc membrane at the photoreceptor outer segment where the phototransduction signalling also takes place (Baseler et al, 2002). Expression of R9AP determines the level of GTPase complex such that any RGS9 produced in excess of R9AP is likely quickly degraded (Martyemyanov et al, 2009). Over-expression of R9AP in the murine rods is sufficient to increase the GTPase activity and to substantially speed their deactivation kinetics as evidenced by the single cell recordings (Krispel et al, 2006). In the cones, the RGS9 expression has been estimated to be ~10-fold higher than that of the rods (Cowan et al, 1998; Zhang et al, 2003). This provides a basis for the ability of the cones to recover quickly from light exposure and thus maintain functional to continuous light stimulus. It also allows cones to respond to more rapid stimulation. Indeed, clinically, patients with delayed deactivation of phototransduction cascade caused by genetic defects in *RGS9* or *R9AP,* or *bradyopsia,* have a profound impairment of cone-mediated vision including day blindness and reduced ability to see moving objects Nishiguchi et al, 2004; Michaelides et al, 2010). Meanwhile, the rod-mediated vision is less affected by the same mutation.

Some macular degeneration conditions, such as age-related macular degeneration (AMD) and inherited macular degeneration conditions also exhibit cone dysfunction but normal or less impaired rod function. Macular degeneration is the leading cause of blindness in the developed world and as its prevalence quadruples in each decade of life the instance of AMD is expected to rise in the coming years as life expectancy increases. Drugs for the treatment of AMD already account for over 1% of the entire drugs budget of the UK's National Health Service. While patients with advanced AMD can be trained to fixate extra-foveally, the low refresh rate and the low bleaching threshold of rod cells limits the quality of resulting vision.

WO2009127705 describes novel therapeutic tools and methods for treating blindness.

WO2013124477 describes methods and compositions for treatment of retinal degeneration diseases.

WO2012167109 describes RPGRIP1 gene therapy for Leber congenital amaurosis.

Lhériteau et al. (Molecular Therapy. 2013; 22(2) pages 265 - 277) describes gene therapy in the RPGRIP1-deficient dog.

Nishiguchi et al. (Nature Communications. 2015; 6(6006) pages 1-10) describes how gene therapy restores vision in rd1 mice after removal of a confounding mutation in Gpr179.

Han et al. (Frontiers in Systems Neuroscience. 2011;5(18) pages 1-8) describes optogenetic control of the non-human primate cortex.

Burns et al. (Physiology. 2010; 25(2), pages 72-84) describes G-protein signalling in living cells.

Pearring et al., (Mol Biol Cell. 2014;25(17):2644-2649) describes how R9AP targeting to rod outer segments is independent of rhodopsin and is guided by the SNARE homology domain.

Sandoval et al., (Invest. Ophthalmol. Vis. Sci. 2006;47(13):853) describes AAV-directed overexpression of R9AP and RGS9-1 in the rat retina.

### SUMMARY OF THE INVENTION

Using mice with absent cone function, we have demonstrated that AAV-mediated over-expression of Rgs9-anchor protein (R9AP), a critical component of GTPase complex that mediates the deactivation of phototransduction cascade, results in desensitization and "photopic shift" of the rod-driven electroretinogram. The treatment enables the rods to respond to brighter light (up to ~2.0 log) than the untreated cells at the expense of scotopic (lower light level) function. Multi-electrode array measurements using the treated retinas showed that the retinal ganglion cells also reflected the "photopic shift" of the rods, by exhibiting graded responses at photopic light levels. Contrast sensitivity function measured by quantifying the head-tracking movements in response to rotating sinusoidal gratings showed an improvement of the sensitivity by up to 25-fold under room light conditions and faster response to repeated stimuli. Furthermore, biochemical measurement of bleachable rhodopsin levels in these mice indicated that the visual cycle was not limiting rod function.

We have also expressed a fast light-driven proton pump, ArchT (Han et al, 2011) in rod photoreceptors. AAV8 particles carrying ArchT-EGFP under control of the Rhodopsin promoter (Rho) were injected subretinally in adult mice. Expression of Rho-ArchT-EGFP was limited to the membrane of rod photoreceptors. Expression of ArchT allowed extremely rapid light responses, while the intrinsic rod response was preserved and was comparable to that observed in non-transduced rod photoreceptors. Overall, ArchT expression did not alter the ability of rod photoreceptors to respond to scotopic stimuli, but it did confer an additional ability to respond with rapid non-bleaching responses to higher levels of illumination. We also found that the transduced rods were able reliably to sustain this fast 'cone-like' transmission, in that ArchT-expressing rods drove sustained retinal ganglion cell (RGC) spiking at high light intensities and at frequencies approaching those of cone photoreceptors. Expression of Rho-ArchT-EGFP in CNGA3-/- and PDE6C-/- mice lacking cone-mediated vision also extended the sensitivity of these mice to bright light stimuli and conferred fast vision on these mice. The maximal frequency of stimuli that ArchT-expressing mice could follow was similar to that of cone photoreceptors.

Together, these results show that, after transduction of healthy rod photoreceptors with genes encoding either light sensitive proteins characteristic of cones or genes encoding molecules that increase the speed of the endogenous rod signalling mechanism, rods behave more like cones and hence can compensate for cone dysfunction. This has implications for the treatment of a number of vision disorders in which cone function is reduced, but at least some healthy rods remain. This contrasts with previous approaches (Busskamp et al, 2010; US Patent Publication No. 2012258530) in which the goal was to restore lost function in cones. Altering function in rods in the manner of the present invention is advantageous in that conditions in which cones are dysfunctional but can be repaired (for example in the early stages of retinal degeneration when photoreceptor function is lost but the photoreceptor-to-bipolar synapse may be intact) are rare, whereas conditions in which cone dysfunction is more severe or advanced and cannot be repaired or where cones are lost entirely, but yet at least some healthy rod photoreceptors remain, are common (see above). Furthermore, this invention enables the creation of a `pseudo-fovea', a small patch of cone-like rods that will improve vision in conditions in which foveal cones have been lost or are dysfunctional.

The invention therefore provides an adeno associated virus (AAV) vector comprising a nucleic acid encoding a gene product that is light-sensitive and/or that modulates endogenous light-sensitive signalling in a photoreceptor cell, for use in a method of improving vision in a patient with cone photoreceptor dysfunction and/or degeneration by introduction of said nucleic acid into healthy rod photoreceptors in the retina of the patient and expression of said gene product therein, such that the range of light intensities to which the rod photoreceptor responds is extended and/or the speed at which the rod photoreceptor responds to light is increased, wherein:
the gene product is ArchT, Jaws (cruxhalorhodopsin), iC1C2, or R9AP;
the capsid of the AAV vector is an AAV8 capsid; and
the nucleic acid is expressed under the control of a rod-specific promoter.

### BRIEF DESCRIPTION OF THE DRAWINGS

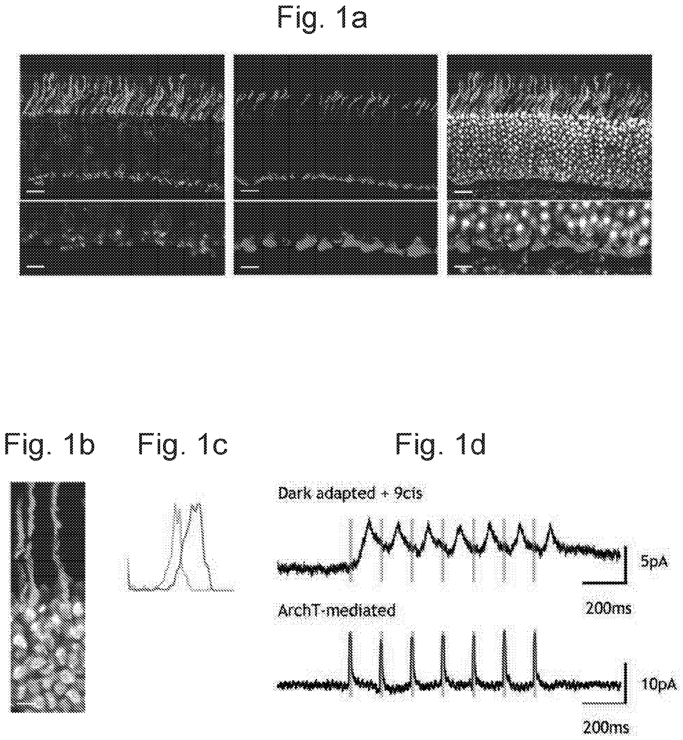

**Figure 1****: Expression of ArchT in rod photoreceptors leads to fast light-driven currents.**
   (a), top panels: AAV8-mediated transduction of ArchT-EGFP (green, see left panels) under control of the Rhodopsin promoter. No overlap is observed with cones (purple: Cone arrestin, see middle panels, white: DAPI, see right panels). Lower panels: specificity of expression can also be observed at the level of synapses. (b) ArchT-EGFP is localized to the membrane of rod photoreceptors, including inner and outer segments. (c) Quantification of fluorescence in ArchT-EGFP-expressing rod terminals (green, left peak) and Cone arrestin positive cone terminals (purple, right peak) shows two distinct bands corresponding to the sub-layer where rod and cone synapses localize respectively (n=22). (d) Single-cell recordings from the cell bodies of ArchT-expressing rod photoreceptors. The currents mediated by the intrinsic rod photo-transduction (upper trace) in response to 10 ms 530-nm light pulses (green, see vertical bars) were preserved. The ArchT-generated currents were faster (lower trace). Scale bars: (a) upper panels: 50 µm; (a) lower panels and (b): 10 um.

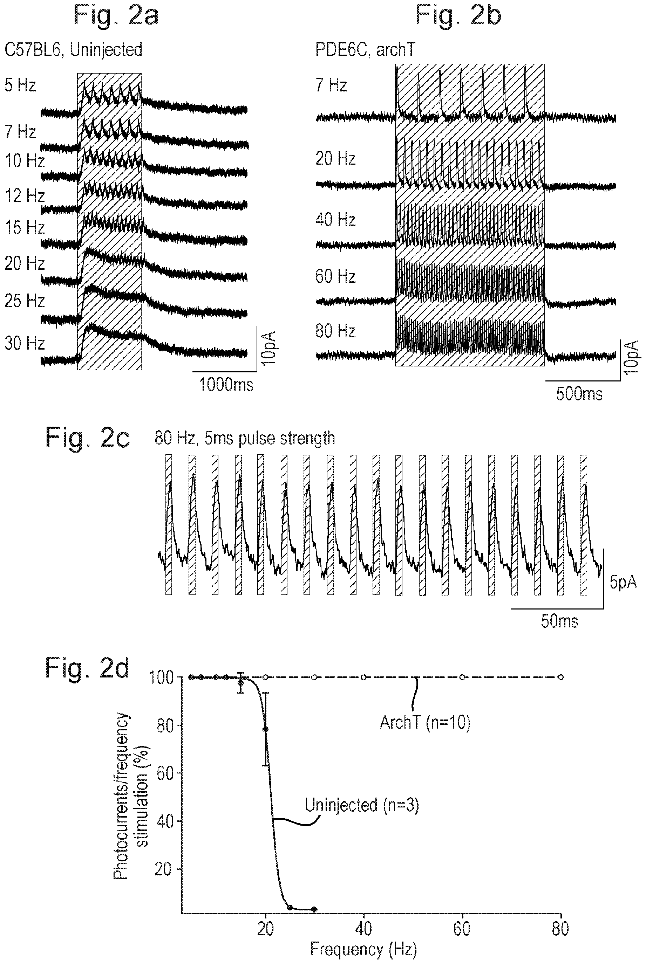

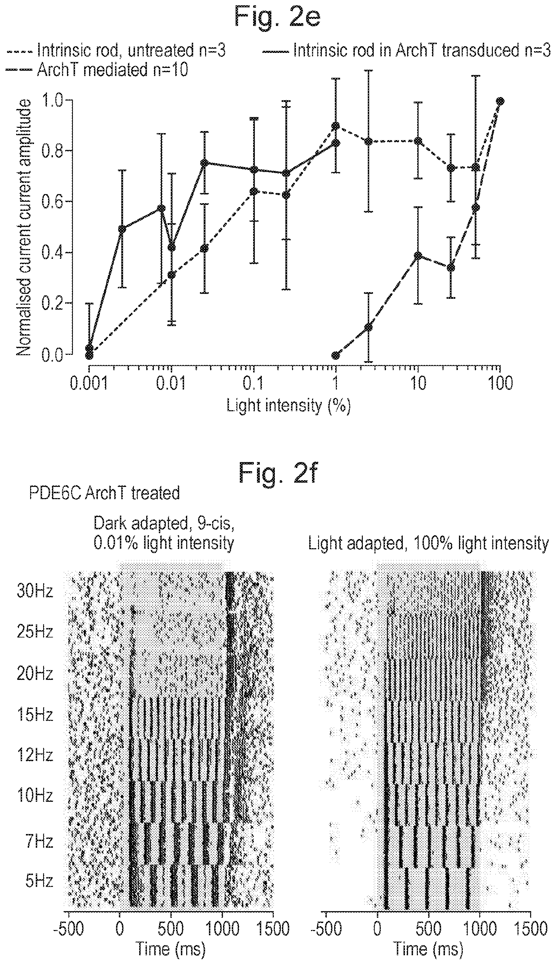

**Figure 2****: ArchT-expression drives high-frequency responses in rods and fast transmission to Retinal Ganglion Cells.**
   (a) Intrinsic rod light-evoked currents in uninjected C57BL6 retinas. (b) ArchT-mediated currents are able to follow much higher stimulation frequencies. (c) Responses are time-locked to stimulus presentation (green vertical bars). (d) ArchT-expressing rods respond to frequency stimulation up to 80 Hz without faltering, whereas intrinsic rod responses drop off at ~20 Hz. (e) Summary data showing that ArchT expression does not alter the intrinsic response of rod photoreceptors, while ArchT responses begin at brighter light levels. (f) Multi-electrode array recordings from PDE6C-/- ArchT-expressing retinas. Intrinsic rod responses failed to elicit reliable Retinal Ganglion Cell spiking above 20 Hz.. On the contrary, ArchT-mediated rod activation drove fast spiking of Retinal Ganglion Cells to levels comparable to cone photoreceptors.

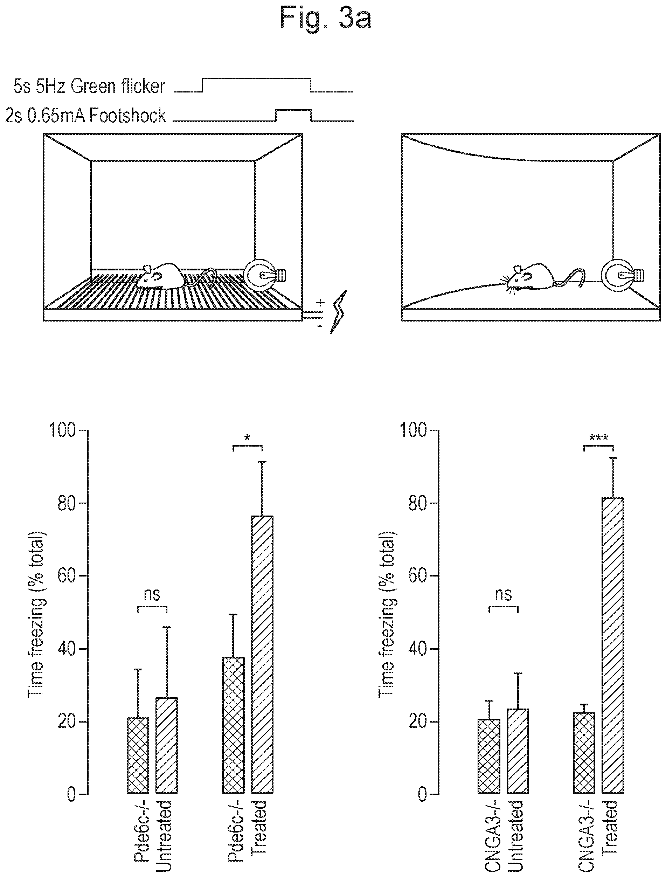

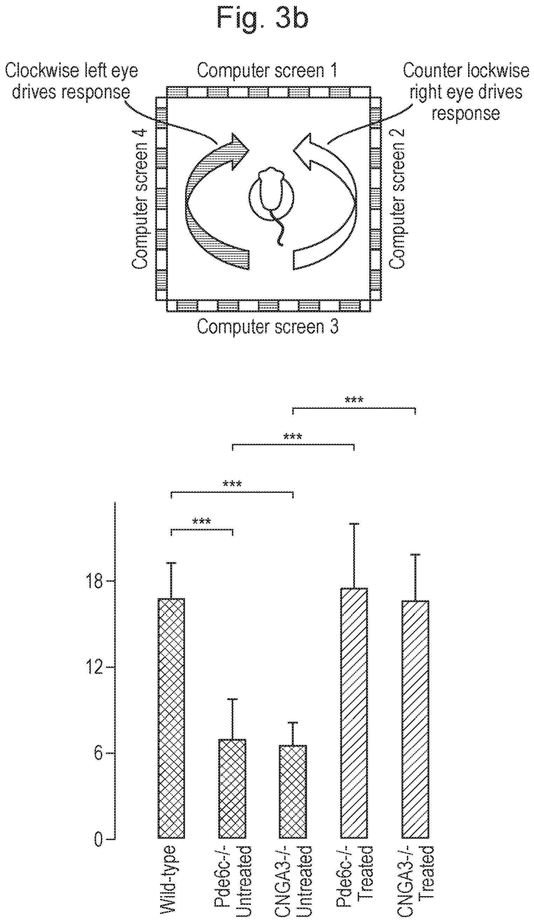

**Figure 3****: ArchT-mediated activation of rods drives behavioural response for fast high light intensity-stimuli.**
   (a) Top panels: schematic for fear conditioning behaviour. Briefly, a visual stimulus was paired with a shock. 24 hours later freezing behaviour was tested in a new context. Bottom panels: uninjected CNGA3^{-/-} and PDE6C^{-/-} mice failed to learn the task (left sets of bars in each graph). However, ArchT expression successfully drove freezing behaviour in mice (right sets of bars in each graph). (b) Optomotor testing. ArchT-expressing mice are able to follow stimuli at frequencies comparable to those reliably followed by cones.

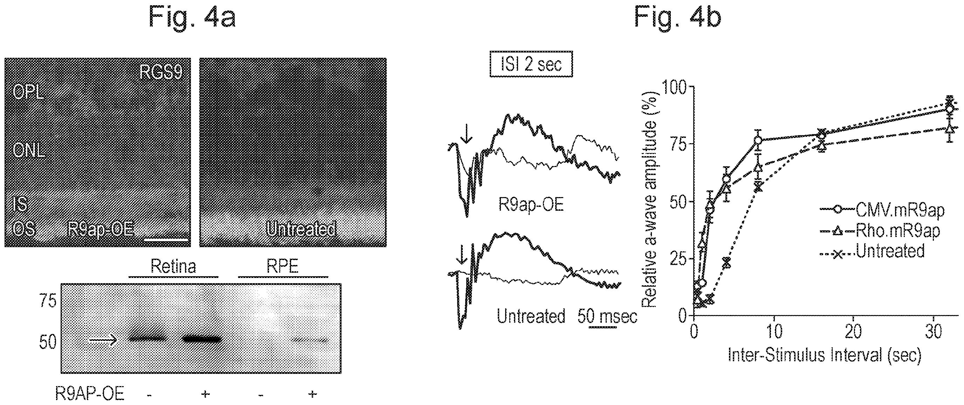

**Figure 4****: AAV-mediated R9AP over-expression in rods and accelerated a-wave deactivation in *Cnga3-*/*-* mice.**
   **A.** Increased RGS9 expression in a *Cnga3-*/*-* eye treated with rRAAV2/8.Rho.mR9ap. Over-expression of R9AP results in increased immunoreactivity toward RGS9 (red) throughout the photoreceptor layer in the treated eye (left) compared to the untreated (right). Western blot shows increased expression of RGS9 both in the retina and retinal pigment epithelium (RPE) in the eye over-expressing R9AP (bottom). A small amount of RGS9 protein was also detected in the RPE of the treated eye. This may reflect "spill over" of the excessive protein contained within the phagocytosed disc membrane. Scale bar indicates 25 µm.
   **B.** Increased speed of a-wave amplitude recovery in the *Cnga3-*/*-* eye treated with rAAV2/8.Rho.mR9ap and rAAV2/8.CMV.mR9ap. Representative ERG tracings for the probe flash (black traces, see traces with peak in the middle of the time course) and for the 2nd flash (red traces) presented at inter-stimulus interval (ISI) of 2 seconds from the treated (top) and untreated (bottom) eyes from the same animal. Note that a second flash yields small a-wave (arrow) is clearly visible in the treated eye, whereas a-wave is not visible (arrow) in the untreated fellow eye. A plot of a-wave recovery at various ISIs in the treated and untreated eyes. The eyes injected with rAAV2/8.CMV.mR9ap (n = 5) or rAAV2/8.Rho.mR9ap (n = 7) have faster recovery kinetics than the untreated eye (n = 5) that is most visible with shorter ISIs. The data is presented as average ± standard error of the mean. OE: over-expression.

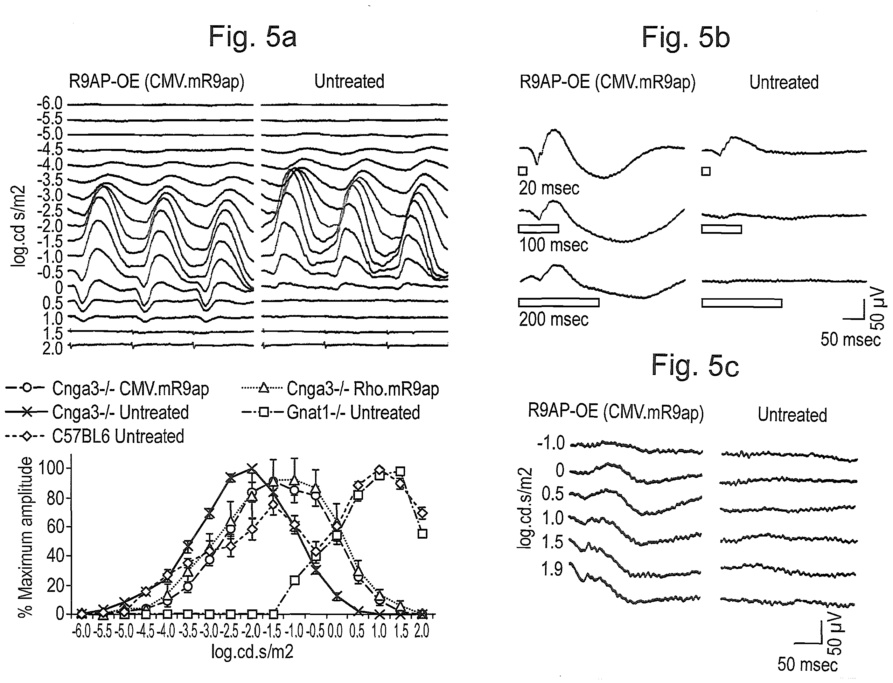

**Figure 5****: Gain of photopic function by rods through over-expression of R9AP in *Cnga3-*/*-* mice.**
   A. Elevation of response threshold and photopic shift of 6Hz ERGs through over-expression of R9AP in rods of *Cnga3-*/*-* mice. Representative 6Hz ERG traces from a *Cnga3-*/*-* mouse in which one eye was treated with rAAV2/8.CMVmR9ap and the other eye was left untreated (top panel). ERG traces are aligned from responses against the dimmest flash (-6.0 log cd.s/m²) to the brightest flash (2.0 log cd.s/m²; bottom) from the top to the bottom at 0.5 log.cd.s/m² step. Note that the lower threshold flash intensity at which the responses emerge is increased, which is coupled with elevated response threshold to brighter flashes. This results in a "photopic shift" of the retinal function in the eye treated with rAAV2/8.CMV.mR9ap. Summary of 6Hz ERG results demonstrating photopic shift of the retinal function following treatment with rAAV2/8.CMV.mR9ap or rAAV2/8.Rho.mR9ap (bottom panel). ERG responses from *Gnat1-*/*-* mice deficient in rod function represents cone-mediated function. Meanwhile, responses from C57BL6 mice are derived from both rod and cone photoreceptors. The data is presented as % amplitude relative to the maximal response and is presented as average ± standard error of the mean. ERGs were recorded from *Cnga3-*/*-* mice treated with rAAV2/8.CMV.mR9ap (Cnga3-/- CMVR9ap; N = 8), *Cnga3-*/*-* mice treated with rAAV2/8.Rho.mR9ap (Cnga3-/- Rho.R9ap; N = 6), *Cnga3-*/*-* mice untreated (Cnga3-/- Untreated; N = 8), *Gnat1-*/*-* mice untreated (Gnat1-/- Untreated; N = 6), and C57BL6 mice untreated (C57BL6 Untreated; N = 6)
   B. Increased retinal responses to long flashes in the *Cnga3-*/*-* eye treated with rAAV2/8.CMV.mR9ap. Open rectangle denotes the duration of the flash. Note that in the eye treated with rAAV2/8.CMVmR9ap, responses are detectable with increased duration of light stimulus. Conversely, the untreated contralateral eye shows little or no response when recorded simultaneously at identical conditions.
   **C.** Gain of retinal function under photopic conditions in the *Cnga3-*/*-* eyes treated with rAAV2/8.CMV.mR9ap. Note that the treated eye shows responses under photopic recording conditions (white background light of 20 cd/m²) whereas the untreated contralateral eye recorded simultaneously remained unresponsive.

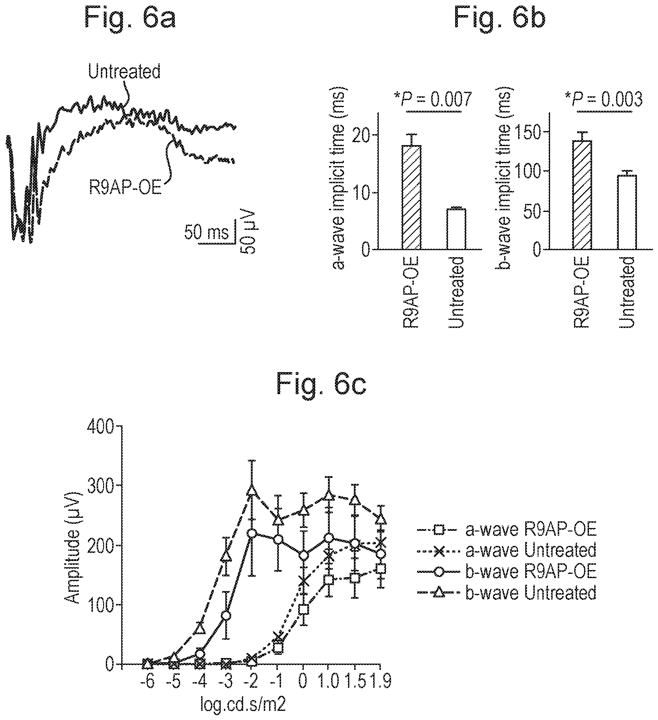

**Figure 6****: Efficient transmission of the altered photoreceptor signal to the bipolar cells in the eyes over-expressing R9AP**
   **A.** Representative ERG traces. ERGs were recorded after rAAV2/8.CMV.mR9ap injection (red trace, lower trace) in a *Cnga3-*/*-* mouse using a saturating flash (1.9 log cd.s/m²). The contralateral eye served as an untreated control (black trace, upper trace).
   **B.** Delayed activation of the bipolar cells to a flash. A-wave and b-wave implicit times were measured from an ERG response to a saturating flash (1.9 log cd.s/m²) in the treated and untreated eyes.
   **C.** Intensity response curve for a-wave and b-wave amplitudes recorded from *Cnga3-*/*-* mice (N=5) with one eye treated with rAAV2/8.CMV.mR9ap (red curves) and the other eye left untreated (black curves). All data are presented as average ± standard error of the mean. OE: over-expression.

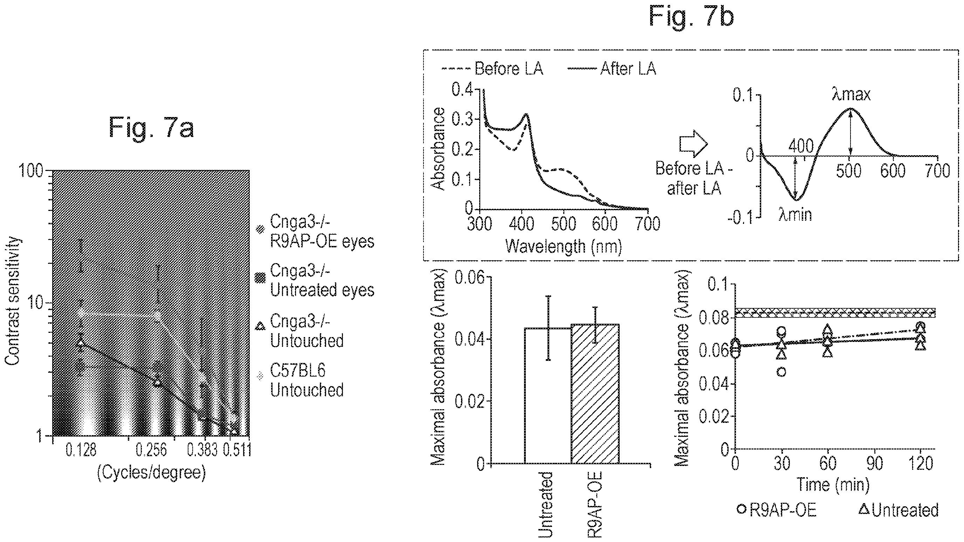

**Figure 7****: A gain of sustainable visual perception following R9AP over-expression in *Cnga3-*/*-* mice.**
   **A.** Improved contrast sensitivity function measured by optokinetic responses. In the *Cnga3-*/*-* mice treated with rAAV2/8.Rho.mR9ap in the left eye, the contrast sensitivity function (CSF) was differentially measured for clockwise (representing treated left eye) and counter-clockwise (representing untreated right eye) head tracking movements against sinusoidal gratings. The CSF for the treated eyes (red curve) was better than that for the untreated eyes (blue curve), which was similar to that for the untouched Cnga3-/- mice (black curve; average of both eyes). Note that the CSF for the treated eye was equivalent to, if not slightly better, to that for untouched wild-type controls (green; average of both eyes). N= 5 for all groups. All data are presented as average ± standard error of the mean. OE: over-expression.
   **B.** Sustained rhodopsin levels after prolonged exposure to Optomotry test. A representative recording of optical absorption of ocular sample using scanning spectrophotometer (left panel inside the green dashed box). Subtracting the absorption of ocular samples measured after (blue trace, top trace between 300 and 400nm) from before (red trace) complete photobleaching showed λmin peaking at ~380 nm corresponding to released photoproducts coupled with λmax peaking at ~500 nm representing the amount of bleachable rhodopsin in the sample (right panel inside the green dashed box). Rhodopsin bleaching speed was assessed by measuring the difference spectrum (λmax) in the fully dilated eyes treated or untreated with rAAV2/8.Rho.mR9ap in Cnga3-/- mice after 5 minutes' exposure to 7.0 mW white light (bottom left; average ± standard error of the mean). Rhodopsin levels was measured also after exposure of the *Cnga3-*/*-* mice to Optomotry test for up to 120 minutes (right bottom; N=3 for each time point) following unilateral injection of rAAV2/8.Rho.mR9ap. The grey area indicates rhodopsin levels (mean ± standard deviation) recorded from untouched *Cnga3-*/*-* mice (N=8) after an overnight dark-adaptation. Dotted line indicates the average. Note that the level of rhodopsin remains stable for at least 2 hours in both the eyes treated with rAAV2/8.Rho.mR9ap and the untreated eyes of *Cnga3-*/*-* mice. Data with error bars were presented as mean ±standard error of the mean.

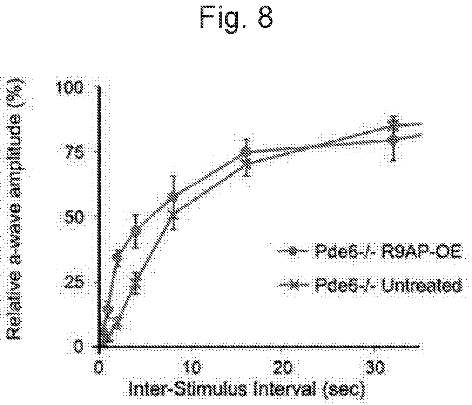

**Figure 8****: Over-expression of R9AP increases the recovery speed of rod photoresponse in *Pde6c-*/*-* mice.**
   The time constant (σ) for 50% recovery of a-wave amplitude was reduced by ~50% in the *Pde6c-*/*-* eyes injected with rAAV2/8.CMV.mR9ap (σ = ∼ 5.75 sec) compared to the untreated contralateral eyes (σ = ∼ 11.46 sec) consistent with accelerated deactivation of phototransduction following the treatment. N = 6. Data with error bars were displayed as mean ±standard error of the mean.

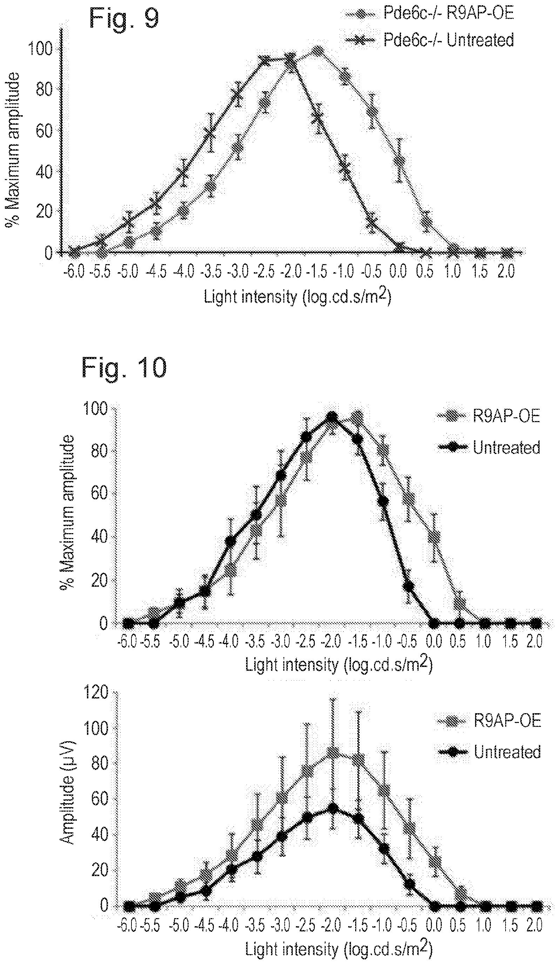

**Figure 9****: Over-expression of R9AP results in "photopic shift" of the intensity-response curve in *Pde6c-*/*-* mice.**
   The eyes injected with rAAV2/8.CMV.mR9ap showed photopic shift of the 6 Hz ERG responses to incremental flash intensities compared to the untreated contralateral eyes in *Pde6c-*/*-* mice (N = 6). The data is presented as % amplitude relative to maximal response and is displayed as average ± standard error of the mean.
**Figure 10****: Sustained effect of R9AP over-expression without clear evidence of retinal degeneration at 5 months post-injection of rAAV2/8.CMV.mR9ap in *Cnga3-*/*-* mice.**
   Response profile normalized against maximum amplitude confirmed the presence of "photopic shift" of the intensity-response curve to 6Hz flashes (top). The same data without normalization showed no evidence of reduction in amplitudes in the treated eye (bottom). N = 5. The data is presented as average ± standard error of the mean.
**Figure 11****: Treatment of wild-type mice with rAAV2/8.Rho.mR9ap showed no obvious effect on 6 Hz ERG intensity-response curve.**
   The eyes treated with rAAV2/8.Rho.mR9ap showed no shift in 6 Hz ERG intensity-response curve compared to that for the untreated contralateral eyes in C57BL6 mice (N = 5). The data is presented as average ± standard error of the mean.
**Figure 12****: No gain of visual perception following R9AP over-expression in C57BL6 mice.**
   In the C57BL6 mice treated with rAAV2/8.Rho.mR9ap only in the left eye, the contrast sensitivity function (CSF) was differentially measured for clockwise (representing treated left eye) and counter-clockwise (representing untreated right eye) head tracking movements to rotating sinusoidal gratings. The CSF for both the treated eyes (pink curve) and the untreated eyes (light blue curve) showed similar results, which was similar to that for the untouched C57BL6 mice (green curve; average of both eyes). N= 5 for all groups. All data are presented as average ± standard error of the mean. OE: over-expression.

### DETAILED DESCRIPTION OF THE INVENTION

A vector comprising a nucleic acid whose expression to produce a gene product, typically a protein, which will effect treatment of an ocular condition as described herein, operably linked to a promoter to form an expression cassette, is described.

### Nucleic Acids and Gene Products

A vector for use in the invention comprises a nucleic acid encoding a gene product that is light-sensitive and/or that modulates endogenous light-sensitive signalling in a photoreceptor cell and makes a rod transduced with the nucleic acid behave more like a cone by extending the range light intensities to which the rod photoreceptor responds is and/or increasing the speed at which the rod photoreceptor responds to light. Thus, the protein may itself be directly light-sensitive, e.g. it may change membrane conductance in rods in a way that results in hyperpolarisation (outward current flow) upon light stimulation. Such proteins will for example be light-sensitive or light-gated G-coupled membrane proteins, ion channels, ion pumps or ion transporters. Light-sensitive proteins are selected from ArchT, Jaws (cruxhalorhodopsin) (Chuong et al, 2014), iC1C2, and R9AP (also known as RGS9BP). The gene products for use in the invention are ArchT, Jaws (cruxhalorhodopsin), iC1C2, and R9AP. Also disclosed herein, the nucleic acid may encode any other gene product that increases the speed of the endogenous rod signalling mechanism. In all of these cases, the sequence may encode a wild-type protein or a mutant or variant or truncated version that retains the activity of the wild-type protein. The nucleic acid may also be codon-optimised for expression in the target cell type. The vector for use in the invention is an adeno-associated virus (AAV) vector, wherein the capsid of the AAV vector is an AAV8 capsid. The nucleic acid for use in the invention is expressed under the control of a rod-specific promoter.

Following expression of the gene product, rods will show stronger and/or faster modulation to light stimuli than non-transduced rods, at higher than usual intensities. Examples include improved modulation strength and/or faster activation/inactivation kinetics. Rods transduced according to the invention will therefore react more strongly and/or quickly to illumination in the mesopic and/or photopic range than non-transduced rods. Preferably, the response of the rods to scotopic illumination conditions is unaffected or not substantially affected, ie the rods gain the ability to respond strongly and/or quickly to brighter light without losing the ability to respond to dim light.

### Promoters and other regulatory elements

In the expression construct, the nucleic acid encoding the gene product is typically operably linked to a promoter. The promoter may be constitutive but will preferably be a photoreceptor-specific or photoreceptor-preferred promoter, more preferably a rod-specific or rod-preferred promoter such as a Rhodopsin (Rho), Neural retina-specific leucine zipper protein (NRL) or Phosphodiesterase 6B (PDE6B) promoter. The promoter region incorporated into the expression cassette may be of any length as long as it is effective to drive expression of the gene product, preferably photoreceptor-specific or photoreceptor-preferred expression or rod-specific or rod- preferred expression.

By a photoreceptor-specific promoter, is meant a promoter that drives expression only or substantially only in photoreceptors, e.g. one that drives expression at least a hundred-fold more strongly in photoreceptors than in any other cell type. By a rod-specific promoter, is meant a promoter that drives expression only or substantially only in photoreceptors, e.g. one that drives expression at least a hundred-fold more strongly in photoreceptors than in any other cell type, including cones. By a photoreceptor-preferred promoter, is meant a promoter that expresses preferentially in photoreceptors but may also drive expression to some extent in other tissues, e.g. one that drives expression at least two-fold, at least five-fold, at least ten-fold, at least 20-fold, or at least 50-fold more strongly in photoreceptors than in any other cell type. By a rod-preferred promoter, is meant a promoter that drives expression preferentially in photoreceptors but may also drive expression to some extent in other tissues, e.g. one that drives expression at least two-fold, at least five-fold, at least ten-fold, at least 20-fold, or at least 50-fold more strongly in photoreceptors than in any other cell type, including cones.

One or more other regulatory elements, such as enhancers, may also be present as well as the promoter.

### Vectors

A vector of the disclosure may be of any type, for example it may be a plasmid vector or a minicircle DNA.

Also disclosed are viral vectors. The viral vector may for example be based on the herpes simplex virus, adenovirus or lentivirus. The viral vector may be an adeno-associated virus (AAV) vector or a derivative thereof. The viral vector derivative may be a chimeric, shuffled or capsid modified derivative.

The viral vector may comprise an AAV genome from a naturally derived serotype, isolate or clade of AAV. The serotype may for example be AAV2, AAV5 or AAV8.

The efficacy of gene therapy is, in general, dependent upon adequate and efficient delivery of the donated DNA. This process is usually mediated by viral vectors. Adeno-associated viruses (AAV), a member of the parvovirus family, are commonly used in gene therapy. Wild-type AAV, containing viral genes, insert their genomic material into chromosome 19 of the host cell. The AAV single-stranded DNA genome comprises two inverted terminal repeats (ITRs) and two open reading frames, containing structural (cap) and packaging (rep) genes.

For therapeutic purposes, the only sequences required *in cis,* in addition to the therapeutic gene, are the ITRs. The AAV virus is therefore modified: the viral genes are removed from the genome, producing recombinant AAV (rAAV). This contains only the therapeutic gene, the two ITRs. The removal of the viral genes renders rAAV incapable of actively inserting its genome into the host cell DNA. Instead, the rAAV genomes fuse via the ITRs, forming circular, episomal structures, or insert into preexisting chromosomal breaks. For viral production, the structural and packaging genes, now removed from the rAAV, are supplied *in trans,* in the form of a helper plasmid. AAV is a particularly attractive vector as it is generally non-pathogenic; the majority people have been infected with this virus during their life with no adverse effects.

The immune privilege of ocular tissue, a result of anatomical barriers and immunomodulatory factors, renders the eye largely exempt from the adverse immunological responses that can be triggered in other tissues by AAV (Taylor 2009).

AAV vectors are limited by a relatively small packaging capacity of roughly 4.8kb and a slow onset of expression following transduction. Despite these minor drawbacks, AAV has become the most commonly used viral vector for retinal gene therapy.

Most vector constructs are based on the AAV serotype 2 (AAV2). AAV2 binds to the target cells via the heparin sulphate proteoglycan receptor. The AAV2 genome, like those of all AAV serotypes, can be enclosed in a number of different capsid proteins. AAV2 can be packaged in its natural AAV2 capsid (AAV2/2) or it can be pseudotyped with other capsids (e.g. AAV2 genome in AAV1 capsid; AAV2/1, AAV2 genome in AAV5 capsid; AAV2/5 and AAV2 genome in AAV8 capsid; AAV2/8).

rAAV transduces cells via serotype specific receptor-mediated endocytosis. A major factor influencing the kinetics of rAAV transgene expression is the rate of virus particle uncoating within the endosome. This, in turn, depends upon the type of capsid enclosing the genetic material (Ibid.). After uncoating the linear single-stranded rAAV genome is stabilised by forming a double-stranded molecule via *de novo* synthesis of a complementary strand. The use of self-complementary DNA may bypass this stage by producing double-stranded transgene DNA. Natkunarajah et al (2008) found that self-complementary AAV2/8 gene expression was of faster onset and higher amplitude, compared to single-stranded AAV2/8. Thus, by circumventing the time lag associated with second-strand synthesis, gene expression levels are increased, when compared to transgene expression from standard single-stranded constructs. Subsequent studies investigating the effect of self-complementary DNA in other AAV pseudotypes (e.g. AAV2/5) have produced similar results. One caveat to this technique is that, as AAV has a packaging capacity of approximately 4.8kb, the self-complementary recombinant genome must be appropriately sized (i.e. 2.3kb or less).

In addition to modifying packaging capacity, pseudotyping the AAV2 genome with other AAV capsids can alter cell specificity and the kinetics of transgene expression.

AAV2/8 is reported to transduce photoreceptors more efficiently than either AAV2/2 or AAV2/5 (Natkunarajah et al. 2008).

The vector for use in the invention comprises an adeno-associated virus (AAV) genome or a derivative thereof. The vector for use in the invention is an adeno-associated virus (AAV) vector comprising a nucleic acid encoding a gene product that is light-sensitive and/or that modulates endogenous light-sensitive signalling in a photoreceptor cell, for use in a method of improving vision in a patient with cone photoreceptor dysfunction and/or degeneration by introduction of said nucleic acid into healthy rod photoreceptors in the retina of the patient and expression of said gene product therein, such that the range of light intensities to which the rod photoreceptor responds is extended and/or the speed at which the rod photoreceptor responds to light is increased, wherein:
the gene product is ArchT, Jaws (cruxhalorhodopsin), iC1C2, or R9AP;
the capsid of the AAV vector is an AAV8 capsid; and
the nucleic acid is expressed under the control of a rod-specific promoter.

An AAV genome is a polynucleotide sequence which encodes functions needed for production of an AAV viral particle. These functions include those operating in the replication and packaging cycle for AAV in a host cell, including encapsidation of the AAV genome into an AAV viral particle. Naturally occurring AAV viruses are replication-deficient and rely on the provision of helper functions in *trans* for completion of a replication and packaging cycle. Accordingly and with the additional removal of the AAV *rep* and *cap* genes, the AAV genome of the vector for use in the invention is replication-deficient.

The AAV genome may be in single-stranded form, either positive or negative-sense, or alternatively in double-stranded form. The use of a double-stranded form allows bypass of the DNA replication step in the target cell and so can accelerate transgene expression.

The AAV genome may be from any naturally derived serotype or isolate or clade of AAV. As is known to the skilled person, AAV viruses occurring in nature may be classified according to various biological systems.

Commonly, AAV viruses are referred to in terms of their serotype. A serotype corresponds to a variant subspecies of AAV which owing to its profile of expression of capsid surface antigens has a distinctive reactivity which can be used to distinguish it from other variant subspecies. Typically, a virus having a particular AAV serotype does not efficiently cross-react with neutralising antibodies specific for any other AAV serotype. AAV serotypes include AAV1, AAV2, AAV3, AAV4, AAV5, AAV6, AAV7, AAV8, AAV9, AAV10 and AAV11, also recombinant serotypes, such as Rec2 and Rec3, recently identified from primate brain. The genome of the vector for use in the invention may be derived from any AAV serotype.. The capsid of the vector for use in the invention is an AAV8 serotype. The genome and the capsid may be derived from the same serotype or different serotypes.

It is preferred that the genome of the vector for use in the invention is derived from AAV serotype 2 (AAV2), AAV serotype 4 (AAV4), AAV serotype 5 (AAV5) or AAV serotype 8 (AAV8). It is most preferred that the genome is derived from AAV2 but other serotypes of particular interest include AAV4, AAV5 and AAV8, which efficiently transduce tissue in the eye, such as the retinal pigmented epithelium. Described are capsids derived from AAV5 or AAV8, especially AAV8. The capsid of the vector for use in the invention is an AAV8 serotype.

Reviews of AAV serotypes may be found in Choi et al (Curr Gene Ther. 2005; 5(3); 299-310) and Wu et al (Molecular Therapy. 2006; 14(3), 316-327). The sequences of AAV genomes or of elements of AAV genomes including ITR sequences, rep or cap genes may be derived from the following accession numbers for AAV whole genome sequences: Adeno-associated virus 1 NC_002077, AF063497; Adeno-associated virus 2 NC_001401; Adeno-associated virus 3 NC_001729; Adeno-associated virus 3B NC_001863; Adeno-associated virus 4 NC_001829; Adeno-associated virus 5 Y18065, AF085716; Adeno-associated virus 6 NC_001862; Avian AAV ATCC VR-865 AY186198, AY629583, NC_004828; Avian AAV strain DA-1 NC_006263, AY629583; Bovine AAV NC_005889, AY388617.

AAV viruses may also be referred to in terms of clades or clones. This refers to the phylogenetic relationship of naturally derived AAV viruses, and typically to a phylogenetic group of AAV viruses which can be traced back to a common ancestor, and includes all descendants thereof. Additionally, AAV viruses may be referred to in terms of a specific isolate, i.e. a genetic isolate of a specific AAV virus found in nature. The term genetic isolate describes a population of AAV viruses which has undergone limited genetic mixing with other naturally occurring AAV viruses, thereby defining a recognisably distinct population at a genetic level.

Examples of clades and isolates of AAV include:
Clade A: AAV1 NC_002077, AF063497, AAV6 NC_001862, Hu. 48 AY530611, Hu 43 AY530606, Hu 44 AY530607, Hu 46 AY530609
Clade B: Hu. 19 AY530584, Hu. 20 AY530586, Hu 23 AY530589, Hu22 AY530588, Hu24 AY530590, Hu21 AY530587, Hu27 AY530592, Hu28 AY530593, Hu 29 AY530594, Hu63 AY530624, Hu64 AY530625, Hu13 AY530578, Hu56 AY530618, Hu57 AY530619, Hu49 AY530612, Hu58 AY530620, Hu34 AY530598, Hu35 AY530599, AAV2 NC_001401, Hu45 AY530608, Hu47 AY530610, Hu51 AY530613, Hu52 AY530614, Hu T41 AY695378, Hu S17 AY695376, Hu T88 AY695375, Hu T71 AY695374, Hu T70 AY695373, Hu T40 AY695372, Hu T32 AY695371, Hu T17 AY695370, Hu LG15 AY695377,
Clade C: Hu9 AY530629, Hu10 AY530576, Hu11 AY530577, Hu53 AY530615, Hu55 AY530617, Hu54 AY530616, Hu7 AY530628, Hu18 AY530583, Hu15 AY530580, Hu16 AY530581, Hu25 AY530591, Hu60 AY530622, Ch5 AY243021, Hu3 AY530595, Hu1 AY530575, Hu4 AY530602 Hu2, AY530585, Hu61 AY530623
Clade D: Rh62 AY530573, Rh48 AY530561, Rh54 AY530567, Rh55 AY530568, Cy2 AY243020, AAV7 AF513851, Rh35 AY243000, Rh37 AY242998, Rh36 AY242999, Cy6 AY243016, Cy4 AY243018, Cy3 AY243019, Cy5 AY243017, Rh13 AY243013
Clade E: Rh38 AY530558, Hu66 AY530626, Hu42 AY530605, Hu67 AY530627, Hu40 AY530603, Hu41 AY530604, Hu37 AY530600, Rh40 AY530559, Rh2 AY243007, Bb1 AY243023, Bb2 AY243022, Rh10 AY243015, Hu17 AY530582, Hu6 AY530621, Rh25 AY530557, Pi2 AY530554, Pi1 AY530553, Pi3 AY530555, Rh57 AY530569, Rh50 AY530563, Rh49 AY530562, Hu39 AY530601, Rh58 AY530570, Rh61 AY530572, Rh52 AY530565, Rh53 AY530566, Rh51 AY530564, Rh64 AY530574, Rh43 AY530560, AAV8 AF513852, Rh8 AY242997, Rh1 AY530556 Clade F: Hu14 (AAV9) AY530579, Hu31 AY530596, Hu32 AY530597, Clonal Isolate AAV5 Y18065, AF085716, AAV 3 NC_001729, AAV 3B NC_001863, AAV4 NC_001829, Rh34 AY243001, Rh33 AY243002, Rh32 AY243003/

The skilled person can select an appropriate serotype, clade, clone or isolate of AAV on the basis of their common general knowledge. It should be understood however that the invention also encompasses use of an AAV genome of other serotypes that may not yet have been identified or characterised. The AAV serotype determines the tissue specificity of infection (or tropism) of an AAV virus. Accordingly, preferred AAV serotypes for use in AAV viruses administered to patients in accordance with the invention are those which have natural tropism for or a high efficiency of infection of rod photoreceptors. The vector for use in the invention is an AAV vector wherein the capsid of the AAV vector is an AAV8 capsid.

Typically, the AAV genome of a naturally derived serotype or isolate or clade of AAV comprises at least one inverted terminal repeat sequence (ITR). Vectors for use in the invention typically comprise two ITRs, preferably one at each end of the genome. An ITR sequence acts in *cis* to provide a functional origin of replication, and allows for integration and excision of the vector from the genome of a cell. Preferred ITR sequences are those of AAV2 and variants thereof. The AAV genome typically comprises packaging genes, such as *rep* and/or *cap* genes which encode packaging functions for an AAV viral particle. The *rep* gene encodes one or more of the proteins Rep78, Rep68, Rep52 and Rep40 or variants thereof. The *cap* gene encodes one or more capsid proteins such as VP1, VP2 and VP3 or variants thereof. These proteins make up the capsid of an AAV viral particle. Capsid variants are discussed below.

Preferably the AAV genome will be derivatised for the purpose of administration to patients. Such derivatisation is standard in the art and the present invention encompasses the use of any known derivative of an AAV genome, and derivatives which could be generated by applying techniques known in the art. Derivatisation of the AAV genome and of the AAV capsid are reviewed in, for example, Choi et al and Wu et al, referenced above.

Derivatives of an AAV genome include any truncated or modified forms of an AAV genome which allow for expression of a Rep-1 transgene from a vector *in vivo.* Typically, it is possible to truncate the AAV genome significantly to include minimal viral sequence yet retain the above function. This is preferred for safety reasons to reduce the risk of recombination of the vector with wild-type virus, and also to avoid triggering a cellular immune response by the presence of viral gene proteins in the target cell.

Typically, a derivative will include at least one inverted terminal repeat sequence (ITR), preferably more than one ITR, such as two ITRs or more. One or more of the ITRs may be derived from AAV genomes having different serotypes, or may be a chimeric or mutant ITR. A preferred mutant ITR is one having a deletion of a *trs* (terminal resolution site). This deletion allows for continued replication of the genome to generate a single-stranded genome which contains both coding and complementary sequences i.e. a self-complementary AAV genome. This allows for bypass of DNA replication in the target cell, and so enables accelerated transgene expression.

In the vectors for use in the invention, the one or more ITRs will preferably flank the expression construct cassette containing the promoter and transgene. The inclusion of one or more ITRs is preferred to aid packaging of the vector of the invention into viral particles. In preferred embodiments, ITR elements will be the only sequences retained from the native AAV genome in the derivative. Thus, a derivative will preferably not include the *rep* and/or *cap* genes of the native genome and any other sequences of the native genome. This is preferred for the reasons described above, and also to reduce the possibility of integration of the vector into the host cell genome. Additionally, reducing the size of the AAV genome allows for increased flexibility in incorporating other sequence elements (such as regulatory elements) within the vector in addition to the transgene.

With reference to the AAV2 genome, the following portions could therefore be removed in a derivative: One inverted terminal repeat (ITR) sequence, the replication (rep) and capsid (cap) genes. However, in some embodiments, including *in vitro* embodiments, derivatives may additionally include one or more *rep* and/or *cap* genes or other viral sequences of an AAV genome.

A derivative may be a chimeric, shuffled or capsid-modified derivative of one or more naturally occurring AAV viruses. Disclosed herein are capsid protein sequences from different serotypes, clades, clones, or isolates of AAV within the same vector. Described is the packaging of the genome of one serotype into the capsid of another serotype i.e. pseudotyping. The invention encompasses the packaging of the genome of one serotype into the capsid of serotype AAV8.

Chimeric, shuffled or capsid-modified derivatives will be typically selected to provide one or more desired functionalities for the viral vector. Thus, these derivatives may display increased efficiency of gene delivery, decreased immunogenicity (humoral or cellular), an altered tropism range and/or improved targeting of a particular cell type compared to an AAV viral vector comprising a naturally occurring AAV genome, such as that of AAV2. Increased efficiency of gene delivery may be effected by improved receptor or co-receptor binding at the cell surface, improved internalisation, improved trafficking within the cell and into the nucleus, improved uncoating of the viral particle and improved conversion of a single-stranded genome to double-stranded form. Increased efficiency may also relate to an altered tropism range or targeting of a specific cell population, such that the vector dose is not diluted by administration to tissues where it is not needed.

Chimeric capsid proteins include those generated by recombination between two or more capsid coding sequences of naturally occurring AAV serotypes. This may be performed for example by a marker rescue approach in which non-infectious capsid sequences of one serotype are cotransfected with capsid sequences of a different serotype, and directed selection is used to select for capsid sequences having desired properties. The capsid sequences of the different serotypes can be altered by homologous recombination within the cell to produce novel chimeric capsid proteins. Chimeric capsid proteins also include those generated by engineering of capsid protein sequences to transfer specific capsid protein domains, surface loops or specific amino acid residues between two or more capsid proteins, for example between two or more capsid proteins of different serotypes.

Shuffled or chimeric capsid proteins may also be generated by DNA shuffling or by error-prone PCR. Hybrid AAV capsid genes can be created by randomly fragmenting the sequences of related AAV genes e.g. those encoding capsid proteins of multiple different serotypes and then subsequently reassembling the fragments in a self-priming polymerase reaction, which may also cause crossovers in regions of sequence homology. A library of hybrid AAV genes created in this way by shuffling the capsid genes of several serotypes can be screened to identify viral clones having a desired functionality. Similarly, error prone PCR may be used to randomly mutate AAV capsid genes to create a diverse library of variants which may then be selected for a desired property.

The sequences of the capsid genes may also be genetically modified to introduce specific deletions, substitutions or insertions with respect to the native wild-type sequence. In particular, capsid genes may be modified by the insertion of a sequence of an unrelated protein or peptide within an open reading frame of a capsid coding sequence, or at the N- and/or C-terminus of a capsid coding sequence.

The unrelated protein or peptide may advantageously be one which acts as a ligand for a particular cell type, thereby conferring improved binding to a target cell or improving the specificity of targeting of the vector to a particular cell population.

The unrelated protein may also be one which assists purification of the viral particle as part of the production process i.e. an epitope or affinity tag. The site of insertion will typically be selected so as not to interfere with other functions of the viral particle e.g. internalisation, trafficking of the viral particle. The skilled person can identify suitable sites for insertion based on their common general knowledge. Particular sites are disclosed in Choi et al, referenced above.

The invention additionally encompasses the provision of sequences of an AAV genome in a different order and configuration to that of a native AAV genome. The invention also encompasses the replacement of one or more AAV sequences or genes with sequences from another virus or with chimeric genes composed of sequences from more than one virus. Such chimeric genes may be composed of sequences from two or more related viral proteins of different viral species.

The vector for use in the invention is an adeno-associated virus (AAV) vector comprising a nucleic acid encoding a gene product that is light-sensitive and/or that modulates endogenous light-sensitive signalling in a photoreceptor cell, for use in a method of improving vision in a patient with cone photoreceptor dysfunction and/or degeneration by introduction of said nucleic acid into healthy rod photoreceptors in the retina of the patient and expression of said gene product therein, such that the range of light intensities to which the rod photoreceptor responds is extended and/or the speed at which the rod photoreceptor responds to light is increased, wherein:
the gene product is ArchT, Jaws (cruxhalorhodopsin), iC1C2, or R9AP;
the capsid of the AAV vector is an AAV8 capsid; and
the nucleic acid is expressed under the control of a rod-specific promoter

Also disclosed is an AAV viral particle comprising a vector described herein. The AAV particles include transcapsidated forms wherein an AAV genome or derivative having an ITR of one serotype is packaged in the capsid of a different serotype. The AAV particles also include mosaic forms wherein a mixture of unmodified capsid proteins from two or more different serotypes makes up the viral envelope. The AAV particle also includes chemically modified forms bearing ligands adsorbed to the capsid surface. For example, such ligands may include antibodies for targeting a particular cell surface receptor.

Also disclosed is a host cell comprising the disclosed vector or AAV viral particle.

Vectors for use in the invention may be prepared by standard means known in the art for provision of vectors for gene therapy. Thus, well established public domain transfection, packaging and purification methods can be used to prepare a suitable vector preparation.

As discussed above, a vector for use in the invention may comprise the full genome of a naturally occurring AAV virus in addition to a promoter or a variant thereof, as disclosed herein. However, commonly a derivatised genome will be used, for instance a derivative which has at least one inverted terminal repeat sequence (ITR), but which may lack any AAV genes such as *rep* or *cap.*

In such embodiments, in order to provide for assembly of the derivatised genome into an AAV viral particle, additional genetic constructs providing AAV and/or helper virus functions will be provided in a host cell in combination with the derivatised genome. These additional constructs will typically contain genes encoding structural AAV capsid proteins i.e. *cap,* VP1, VP2, VP3, and genes encoding other functions required for the AAV life cycle, such as *rep.* The selection of structural capsid proteins provided on the additional construct will determine the serotype of the packaged viral vector.

A particularly preferred packaged viral vector for use in the invention comprises a derivatised genome of AAV2 in combination with AAV8 capsid proteins.

As mentioned above, AAV viruses are replication incompetent and so helper virus functions, preferably adenovirus helper functions will typically also be provided on one or more additional constructs to allow for AAV replication.

All of the above additional constructs may be provided as plasmids or other episomal elements in the host cell, or alternatively one or more constructs may be integrated into the genome of the host cell.

### Pharmaceutical Compositions, Dosages and Treatments

The vector for use in the invention can be formulated into pharmaceutical compositions. These compositions may comprise, in addition to the vector, a pharmaceutically acceptable excipient, carrier, buffer, stabiliser or other materials well known to those skilled in the art. Such materials should be non-toxic and should not interfere with the efficacy of the active ingredient. The precise nature of the carrier or other material may be determined by the skilled person according to the route of administration, i.e. here direct retinal, subretinal or intravitreal injection.

The pharmaceutical composition is typically in liquid form. Liquid pharmaceutical compositions generally include a liquid carrier such as water, petroleum, animal or vegetable oils, mineral oil or synthetic oil. Physiological saline solution, magnesium chloride, dextrose or other saccharide solution or glycols such as ethylene glycol, propylene glycol or polyethylene glycol may be included. In some cases, a surfactant, such as pluronic acid (PF68) 0.001% may be used.

For injection at the site of affliction, the active ingredient will be in the form of an aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection, Hartmann's solution. Preservatives, stabilisers, buffers, antioxidants and/or other additives may be included, as required.

For delayed release, the vector may be included in a pharmaceutical composition which is formulated for slow release, such as in microcapsules formed from biocompatible polymers or in liposomal carrier systems according to methods known in the art.
The vectors for use in the invention can be packaged into a kit.

In general, direct retinal, subretinal or intravitreal delivery of the disclosed vectors, typically by injection, is preferred. Delivery to the retinal, subretinal space or intravitreal space is thus preferred. Vectors may also be introduced into rod photoreceptors *in vitro* followed by cell transplantation into the retina

The vectors for use in the invention can also be used in combination with any other therapy for the treatment or prevention of vision disorders. For example, they may be used in combination with known treatments that employ VEGF antagonists, eg anti-VEGF antibodies such as Bevacizumab or Ranibizumab or soluble receptor antagonists such as Aflibercept, for the treatment of AMD or other eye disorders as discussed herein.

Dosages and dosage regimes can be determined within the normal skill of the medical practitioner responsible for administration of the composition. The dose of a vector for use in the invention may be determined according to various parameters, especially according to the age, weight and condition of the patient to be treated; the route of administration; and the required regimen. Again, a physician will be able to determine the required route of administration and dosage for any particular patient.

A typical single dose is between 10¹⁰ and 10¹² genome particles, depending on the amount of retinal tissue that requires transduction. A genome particle is defined herein as an AAV capsid that contains a single stranded DNA molecule that can be quantified with a sequence specific method (such as real-time PCR). That dose may be provided as a single dose, but may be repeated for the fellow eye or in cases where vector may not have targeted the correct region of retina for whatever reason (such as surgical complication). The treatment is preferably a single permanent treatment for each eye, but repeat injections, for example in future years and/or with different AAV serotypes may be considered.

### Treatments

Vectors for use in the invention may be used to treat any ocular condition in which there is dysfunction, degeneration or absence of cones but at least some healthy rods remain. Cone function may be wholly or partially missing, e.g. at least 10%, at least 25%, at least, at least 50%, at least 75%, at least 80%, at least 90% or more missing. Healthy rods are rods that are capable of performing normal or partial, e.g. at least 10%, at least 25%, at least, at least 50%, at least 75% or at least 90% of normal rod function in terms of perception of light at scotopic levels.

Vectors for use of the invention can be used in a method for treating conditions including macular degeneration, achromatopsia and Leber congenital amaurosis. The macular degeneration may be age-related macular degeneration (AMD), for example wet or neovascular AMD or geographic atrophy, an inherited macular degeneration condition or an inherited cone dystrophy. In some embodiments, the invention will result in the creation of a `pseudo-fovea', a small patch of cone-like rods that improves vision in conditions in which foveal cones have been lost or are dysfunctional.

In general, patients to be treated with vectors will be human patients. They may be male or female and of any age.

The following Examples illustrate the invention.

### EXAMPLES

### Example 1 - Methods for ArchT experiments.

### Animals

Wild-type mice (C57BL/6J) were purchased from Harlan Laboratories (Blackthorn, UK). CNGA3-/- and PDE6C-/- mice were bred in house. All mice were maintained under cyclic light (12 h light-dark) conditions; cage illumination was 7 foot-candles during the light cycle. All experiments were approved by the local Institutional Animal Care and Use Committees (UCL, London, UK) and conformed to the guidelines on the care and use of animals adopted by the Society for Neuroscience and the Association for Research in Vision and Ophthalmology (Rockville, MD).

### Plasmid constructions, viral production and injection procedure

The transgene construct (ArchT-EGFP) was kindly provided by Prof Ed Boyden (MIT, USA) and contains the cDNA sequence of the ArchT gene fused to the fluorescent protein EGFP. The plasmids were packaged into AAV8 to generate recombinant AAV viral vectors, AAV8.hRho.ArchT-EGFP. Recombinant AAV8 vector was produced through a triple transient transfection method as described previously. The plasmid construct, AAV serotype-specific packaging plasmid and helper plasmid were mixed with Polyethylenimine (Polysciences Inc.) to form transfection complexes, which were then added to 293T cells and left for 72h. The cells were harvested, concentrated and lysed to release the vector. AAV8 was purified using AVB Sepharose columns (GE Healthcare). Both were washed in 1X PBS and concentrated to a volume of 100-150 µL. Viral particle titres were determined by comparative dot-blot DNA prepared from purified viral stocks and defined plasmid controls. Purified vector concentrations used for all experiments were 5 × 10¹² viral particles/ml. Subretinal injections were performed as described previously by our group and consisted of double injections of 2ul each.

### Immunohistochemistry

Animals were euthanized, the eyeballs enucleated and cornea, lens and iris removed. For retinal sections, the eyecups were fixed in 4% paraformaldehyde (PFA) for 1 hour at room temperature, before embedding in optimal cutting temperature (OCT) medium. 30µm cryosections were cut in sagittal orientation, rinsed with PBS and blocked in 10% normal goat serum (NGS), 3% bovine serum albumin (BSA) and 0.1% Triton-X100. The respective samples were incubated with primary antibodies in block solution at 4°C overnight using rabbit anti-cone arrestin (diluted 1:500). Following PBS washes, the respective combination of secondary antibodies (all diluted 1:500, life technologies) including goat anti rabbit Alexa Fluor 546 (#A11035), goat anti mouse Alexa Fluor 633 (#A21052) and streptavidin, Alexa Fluor 633 conjugate (#S21375) were used to label the samples before these were counterstained with DAPI and mounted with DAKO fluorescent mounting media (DAKO, S3023, Denmark). Images were acquired by confocal microscopy (Leica DM5500Q).

### Single photoreceptor suction recordings

Animals were dark adapted for 12h prior to the start of experiments. Mice were administered an overdose of ketamine-dormitor anaesthetic mix via the intra- peritoneal cavity, to induce terminal surgical-plane anaesthesia. Mice were then sacrificed by cervical dislocation and enucleated. Eyes were dissected under dim, far-red illumination. Isolated retinas were imbedded in 1% low melting agarose solution and then cut using a vibrotome (leica) into 230um thick en face slices. Slices were mounted in a recording chamber and perfused with carbogen (95% O₂ 5% CO₂) saturated Ames medium containing 100µm 9-cis retinal (Sigma) and 0.2% BSA (Sigma). The temperature of the perfusion solution was maintained at 37°C using an in-line heating element under feedback control (Scientifica). Very low resistance (1-2MΩ ) patch pipettes were made from filamented boroscilicate glass capillaries (Harvard Apparatus Ltd) using a Narishige PC-10 vertical puller. Pipettes were filled with external solution, mounted onto the headstage and a small pressure applied across the tip (~30mbar). Using infrared illumination and a microscope to aid visualisation the pipette was placed onto the surface of the retina, and then lowered ~50um into the slice, until photoreceptor segments appeared intact and neatly arranged. Slight negative pressure was applied across the pipette tip as it was advanced slowly through the retinal tissue, using a 100ms, 10mV test pulse to monitor resistance across the pipette tip. When resistance increased to ~20-30MΩ light evoked responses were tested. Light stimuli from an LED light-source (peak wavelength 530nm) coupled to a liquid light guide, were delivered through the microscope objective (Olympus). Neutral density filters were used to precisely control the intensity of the light stimulus. Light stimuli consisted of square wave pulses programmed using P-Clamp software (Molecular Devices), and delivered via a DAC board (Axon Instruments) in conjunction with an LED driver (Thorlabs). Electrophysiological recordings were carried out using a Multiclamp 700B amplifier (Molecular Devices). Data was digitized at 20kHz.

### MEA recordings

Animals were dark adapted for 12h prior to the start of experiments. Mice were administered an overdose of ketamine-dormitor anaesthetic mix via the intra- peritoneal cavity, to induce terminal surgical-plane anaesthesia. Mice were then sacrificed by cervical dislocation and enucleated. Eyes were dissected in carbogen (95% Oxygen, 5% Carbon Dioxide) saturated Ames medium (Sigma), under dim red light. The cornea and lens were removed, with care taken to remove as much vitreous from the surface of the retina as possible. The RPE was separated from the retina and a flat petal 1-3 mm across was cut away from the retinal 'cup'. This retinal petal was placed ganglion-cell side down on the surface of the multielectrode array, and a circular harm constructed from unreactive platinum wire (Sigma) and nylon was used to keep the petal in position. Throughout recordings, the tissue was perfused with carbogen saturated Ames medium (Sigma), maintained at a temperature of 36.5 degrees Celsius. For recordings that included scotopic or mesopic conditions, the perfusion medium was made up to include 9-cis retinal (Sigma), at a concentration of 100µM, in 0.2% BSA (Sigma). A perforated 60-electrode recording array, consisting of tungsten electrodes spaced 100 µm apart (Multi Channel Systems) was used to record ganglion cell extracellular potentials. Voltage changes were amplified and digitized at 50kHz by an MC Card system, using MC Rack software (MultiChannel Systems).

### Electrophysiological data analysis

Electrophysiological data was analysed using custom-written macros in IgorPro 6. Synaptic currents and potentials were detected using an amplitude threshold algorithm where the threshold for event detection was set at 2 times the standard deviation of the baseline noise (typically about 10pA). Detected currents and potentials were verified manually through careful inspection of all electrophysiological data.

### Fear conditioning

Mice were trained and tested using a commercially available fear conditioning system (Med Associates). To ensure blind conditions, the experimenter performing the training and testing was always blind to the strain of mouse and treatment conditions. Briefly, the setup consisted of a conditioning chamber (20x30 cm) with a stainless steel grid floor placed inside a sound-attenuating cubicle. Mouse behaviour was monitored constantly during training and testing by means of a built-in infrared digital video camera (30 frames/s acquisition rate) and infrared illumination. Video Freeze software (Med Associates) was used to control delivery of the light stimulus and shock. The light stimulus consisted of a single LED (530 nm, Thorlabs) 5 Hz 50ms full-brightness flicker generated via an Arduino interface (Arduino Software) positioned on a side panel of the conditioning chamber. To ensure that the context in which training and testing took place were different, floor and curved wall panels were inserted into the chamber for the testing session. A background white light was used to reduce chances of rod activation and pupils were dilated with Tropicamide drops to increase the amount of light reaching the mouse retina.

Mice were placed inside the chamber and underwent one conditioning session, consisting of 6 pairings of a 5 s, light stimulus that co-terminated with a 2 s, 0.65 mA foot shock. Inter-trial interval was pseudo-randomized (average interval 90 s). Following the training session mice were returned to the home cage. 24 hours after training, mice were tested for visually cued memory recall. Mice were placed in the test chamber and monitored for a total of 360 s. The conditioning light stimulus was presented continuously for the last 120 s of the test session. All data was acquired and scored automatically by VideoFreeze software (Med Associates). Briefly, the software is calibrated before placing the animal in the chamber. The software then measures the pixel changes that take place between every video frame. The motion threshold was set to be as low as possible (20 motion index units), and the continuous freezing count was set to the frame rate to ensure the most sensitive read-out of motion. To assess light cued memory recall the percentage time of freezing behaviour was averaged for the two minutes immediately prior to and following the light stimulus onset. Statistical significance was assessed with a one-way ANOVA. Results are presented as mean ± S.E.M.

### Optomotry

Visual acuities were measured by observing the optomotor responses of mice to rotating sinusoidal gratings (OptoMotry, Cerebral Mechanics). The protocol used yields independent measures of the acuities of right and left eyes based on the unequal sensitivities of the two eyes to pattern rotation as only motion in the temporal-to-nasal direction evokes the tracking response. As a result, the right and the left eyes are most sensitive to counter-clockwise (CCW) and clockwise (CW) rotations, respectively. Stimuli of different temporal frequency were used to determine the threshold at which a response was present. A double-blind two-alternative forced choice procedure was employed, in which the observer was 'blind' to the direction of pattern rotation, to whether it was an ArchT-treated or untreated *CNGA3-*/*- or PDE6C-*/*-* mouse or age-matched wild-type control animal (C57BL6). Visual acuity was measured in both eyes of the tested animal and averaged or separately analyzed for each eye after 4 trials were conducted on 4 separate days. The measurement was carried out on injected mice 3-10 weeks after treatment together with age-matched isogenic controls.

### Example 2 - ArchT expression in rod photoreceptors confers the ability to respond with rapid non-bleaching responses

Rod-mediated vision is optimized for low light levels, including single photon detection. However, rods cannot match the rapid onset and recovery of cone responses to light (Fu et al., 2007, Pugh et al., 1999). This functional difference, useful to ensure reliable vision in different environments, becomes debilitating when cone-mediated vision is lost in conditions such as in age-related macular degeneration, when the densely packed cones in the fovea degenerate (de Jong 2006). It was investigated whether if rods could respond and recover more quickly to stimuli, this would help alleviate the functional impairment caused by the loss of cones.

A fast light-driven proton pump (ArchT) (Han et al., 2011) was expressed in rod photoreceptors. AAV8 particles carrying ArchT-EGFP under control of the Rhodopsin promoter (Rho) were injected subretinally in adult mice. Expression of Rho-ArchT-EGFP was limited to the membrane of rod photoreceptors (Figures1a-b). Synaptic terminals of rods expressing Rho-ArchT-EGFP and cones could be easily distinguished following immunohistochemistry (Figure 1c). Quantitative PCR on a sorted population of cones confirmed that Rho-ArchT-EGFP expression was specific to the rod population and no evident sign of toxicity was observed up to 6 months following AAV8 injection. Expression of ArchT allowed extremely rapid light responses, while the intrinsic rod response was preserved and was comparable to that observed in non-transduced rod photoreceptors (Figure 1d). Light-evoked currents recorded from ArchT-expressing rods demonstrated considerably faster kinetics than the intrinsic rod currents in all mouse models tested (Figure 2a-b). These kinetics allowed light evoked currents to be modulated up to 80 Hz, far above the limits of both rods, which faltered at ~20 Hz (Figure 2a-c), and of cones (Fu et al., 2007).

Surprisingly, ArchT expression did not alter the properties of rod photoreceptors while conferring the ability to respond with rapid non-bleaching responses (Figure 2e).

### Example 3 - ArchT-expressing rods drive sustained RGC spiking at high light intensities and at frequencies approaching those of cone photoreceptors

It was next investigated whether the circuitry driven by rods would be able to follow faster-than-normal rod-driven vision. Rod and cone pathways present some similarities and some striking differences and it is therefore not clear whether the rod circuitry can reliably sustain fast 'cone-like' transmission (Wässle et al., 2004). Rods have been shown to contact OFF 'cone' bipolar cells directly (Soucy et al., 1998 and Hack et al., 1999) and paired-pulse stimulation suggests that this alternative pathway may be as fast as the cone-to-OFF-bipolar one (Li et al., 2010). However, it is not clear how sustained this response can be and whether rod (ON) bipolar cells can also sustain fast transmission. Furthermore, rod synaptic terminals have different size and ultrastructural organization compared to cones and rod bipolar cells do not contact Retinal Ganglion Cells (RGCs) directly but only through a pathway involving All amacrine cells (Wässle et al., 2004). To investigate the maximal speed that the rod pathway can achieve, multi-electrode recordings from RGCs in mouse models lacking cone function were performed, to isolate rod-mediated RGC output. Rod-driven responses in RGCs in non-transduced retinas were bleached at high light levels and could not follow stimulation frequencies higher than ~20 Hz (Figure 2f).

On the contrary, ArchT-expressing rods drove sustained RGC spiking at high light intensities and at frequencies approaching those of cone photoreceptors (Figure 2f).

### Example 4 - expression of Rho-ArchT-EGFP extended the sensitivity of mice lacking cone-mediated vision to bright light stimuli

For this faster-than-normal rod vision to be useful, it was reasoned that mice should be able to use ArchT-mediated currents to reliably respond to bright and fast stimuli. CNGA3^{-/-} and PDE6C^{-/-} mice lacking cone-mediated vision (Biel et al., 1999 and Change et al., 2009) failed to learn a fear conditioning paradigm where bright light stimuli were paired and co-terminated with a mild foot shock (Figure 3a). However, expression of Rho-ArchT-EGFP extended the sensitivity of these mice to bright light stimuli, allowing learning of the association between visual stimulus and shock (Figure 3a). Finally, it was tested whether ArchT expression conferred fast vision to CNGA3^{-/-}and PDE6C^{-/-} mice. Assessment of the speed of vision by means of Optomotor testing (Umino et al., 2008) showed that ArchT-expressing mice were able to follow stimuli faster than mice that did not undergo subretinal viral injections and mice that received a GFP-only vector (Figure 3b). The maximal frequency of stimuli that ArchT-expressing mice could follow was similar to that of cone photoreceptors (Figure 3b).

Together, these results show that rods can be driven faster than by their intrinsic photo-transduction cascade and that rod-driven circuits can sustain a faster signalling. Importantly, synaptic release from rods does not require large voltage fluctuations, but small currents can instead cause sufficient voltage variations to significantly alter their synaptic transmission (Cangiano et al., 2012). This extends the use of the Invention to light levels several-fold lower than the average light levels required for optogenetic manipulation of activity in most other neurons (Han et al., 2011).

### Example 5 - Methods for RPAP experiments

### Animals

C57BL6 (Harlan, UK), *Cnga3-*/*-* (J.R. Heckenlively, University of Michigan), *Pde6c*/- (J.R. Heckenlively, University of Michigan, MI) (Chang et al., 2009), and *Gnat1-*/*-* (J. Lem, Tufts University School of Medicine, MA) (Calvert et al., 2000) mice were maintained in the animal facility at University College London. Adult male and female animals were 6-12 weeks old at the time of viral injection and were used for experiments at least 2 weeks after the injection to allow for a sufficient expression of R9AP. All the mice used were between ages of 2 to 6 months and were age matched between groups of a given experiment. All experiments have been conducted in accordance with the Policies on the Use of Animals and Humans in Neuroscience Research and with the ARVO Statement for the Use of Animals in Ophthalmic and Vision Research. Animals were kept on a standard 12/12 hour light-dark cycle

### Plasmid constructions and production of recombinant AAV8

The murine *R9ap* cDNA was PCR amplified from murine retinal cDNA using primers which have been designed to encompass the whole of the coding region. The *R9ap* cDNA was cloned between the promoter (CMV promoter or bovine rhodopsin promoter) and the SV40 polyadenylation site. These plasmids were used to generate two pseudotyped AAV2/8 viral vectors, rAAV2/8.CMV.mR9ap and rAAV2/8.Rho.mR9ap, as described below.

Recombinant AAV2/8 vector was produced through a triple transient transfection method as described previously (Gao et al., 2002). The plasmid construct, AAV serotype-specific packaging plasmid and helper plasmid were mixed with polyethylenimine to form transfection complexes which was then added to 293T cells and left for 72 h. The cells were harvested, concentrated and lysed to release the vector. The AAV2/8 was purified by affinity chromatography and concentrated using ultrafiltration columns (Sartorius Stedim Biotech, Goettingen, Germany), washed in PBS and concentrated to a volume of 100-150 µl. Viral particle titres were determined by dot-blot or by real-time PCR. Purified vector concentrations used were 1-2 × 10¹² viral particles/ml.

### Electroretinogram (ERG)

ERGs were recorded from both eyes after mice were dark adapted overnight using a commercially available system (Espion E2, Diagnosys LLC, Lowell, MA). The animals were anesthetized with an intraperitoneal injection of a 0.007 ml/g mixture of medetomidine hydrochloride (1 mg/ml), ketamine (100 mg/ml), and water at a ratio of 5:3:42 before recording. Pupils were fully dilated using 2.5% phenylephrine and 1.0% tropicamide. Midline subdermal ground and mouth reference electrodes were first placed, followed by positive silver electrodes that were allowed to lightly touch the center of the corneas under dim red illumination. A drop of Viscotears 0.2% liquid gel (Dr. Robert Winzer Pharma/OPD Laboratories, Watford, UK) was placed on top of the positive electrodes to keep the corneas moistened during recordings and the mouse was allowed to further dark-adapt for 5 minutes. Bandpass filter cutoff frequencies were 0.312 Hz and 1000 Hz. Recovery speed of photoresponse was measured using paired flash paradigm where pairs of flashes with identical saturating intensity (1.8 log.cd.s/m²) separated by various inter-stimulus intervals (ISI; 0.5, 1, 2, 4, 8, 16, 32, 64 sec) were presented. In this paradigm, the 1^{st} flash would completely suppress the electric responses of rod mechanisms which allow observation of the speed of functional recovery of the rod function by presenting 2^{nd} flash with different ISI. Sufficient amount of time (150 sec) were provided between pairs of flashes to allow full recovery of the 1^{st} flash. Then the recovery of a-wave amplitude observed should reflect the speed of deactivation of the rods in animals devoid of cone function since the flash should only bleach a fraction (0.02%) of the rhodopsin (Lyubarsky et al., 2004 and Weymouth, A.E. & Vingrys 2008). Scotopic 6 Hz flicker intensity series were performed as previously reported with a few modifications (Seeliger et al., 2001). 17 steps of flash intensities were used ranging from -6 to 2 log.cd.s/m² each separated by 0.5 log unit. For each step, after 10 seconds of adaptation, 600 msec sweeps were averaged 20 times using the same flash condition. Series of dark-adapted responses were also obtained using longer flashes with durations of 20, 100, and 200 msec all at 83.3 cd/m². Standard single flash scotopic recordings were obtained from dark-adapted animals at the following increasing light intensities: -6, -5, -4, -3, -2, -1, 0, 1.0, 1.5, and 1.9 log.cd.s/m². Photopic flash recordings were performed following 5 min light adaptation intervals on a background light intensity of 20 cd/m², which was also used as the background light for the duration of the recordings. Photopic light intensities used were -2, -1, 0, 1, 1.5, and 1.9 log.cd.s/m².

### Histology

Six weeks after unilateral subretinal injection of rAAV2/8.Rho.mR9ap, both eyes from a *Cnga3-*/*-* mouse were quickly removed and snap frozen in liquid nitrogen. After cryoembedding the eye in OCT (RA Lamb, Eastborne, UK), the eyes were cut as transverse sections 15 µm thick and were air-dried for 15 - 30 min. For immunohistochemistry, sections were pre-blocked in PBS containing normal donkey serum (2%), bovine serum albumin (2 %) 1 hr before being incubated with anti-RGS9 antibody (1:500; Santa Cruz Biotechnology, SantaCruz, CA) for 2 hours at room temperature. After rinsing 2 x 15 min with PBS, sections were incubated with the appropriate Alexa 546-tagged secondary antibody (Invitrogen, Carlsbad, CA) for 2 hrs at room temperature (RT), rinsed and counter-stained with Hoechst 33342 (Sigma-Aldrich, Gillingham, UK). Retinal sections were viewed on a confocal microscope (Leica TCS SP2, Leica Microsystems; Wetzlar, Germany).

### Western blotting

The eyes from a *Cnga3-*/*-* mouse 4 weeks after unilateral subretinal injection of rAAV2/8.Rho.mR9ap were collected. After separating the neural retina from the RPE/choroid/sclera complex, tissues were homogenized in RIPA buffer and left on ice for 20 minutes. The samples were centrifuged at 16,000 g for 30 minutes at 4 °C and stored in -20°C until use. Western blotting was carried out using known protocols.

### Optomotor responses and contrast sensitivity function

Contrast sensitivities and visual acuities of treated and untreated eyes were measured by observing the optomotor responses of mice to rotating sinusoidal gratings (OptoMotry^{™}, Cerebral Mechanics, Lethbridge, AB Canada). The protocol used yields independent measures of the acuities of the right and the left eyes based on unequal sensitivities of the two eyes to pattern rotation: the right and the left eyes are driven primarily by counter-clockwise and clockwise rotations, respectively (Douglas et al., 2005). A mouse was placed on a small island isolated from the floor in a closed space surrounded by 4 monitors with rotating sinusoidal grating with a mean illuminance of 62 cd/m². A double-blind two-alternative forced choice procedure was employed, in which the observer was 'blind' to the direction of pattern rotation, to whether it was a treated or untreated *Cnga3-*/*-* mouse or age-matched wild-type control animal (C57BL6). The contrast sensitivity measured at 0.128, 0.256, 0.383, 0.511 cycles/degree presented at 6 Hz was defined as 100 divided by the lowest percent contrast yielding a threshold response. Both eyes of each mouse were tested four times on independent days. The data was projected on to a Campbell-Robson Contrast Sensitivity Chart with sinusoidal gratings representing relative spatial frequencies.

### Rhodopsin measurement

After fully dark-adapting the mice overnight, the mice were anaesthetized and the pupils were fully dilated to assess the speed of visual pigment bleaching. Then the mice were placed in a light box with a light source (7.0 mW) directly illuminating the eye for 5 minutes before the eyes were collected. In another experiment, mice were exposed to an identical condition as that for measuring contrast sensitivity for various durations (0, 30 60, 120 minutes). The mouse eyes were removed at each time point and placed in 250 µl of phosphate buffered saline and snap frozen in liquid nitrogen in a light tight tube and kept at -20 °C until use. Some eyes were collected in the dark under red illumination after overnight dark-adaptation of the mice. Spectrophotometric measurement of rhodopsin were performed as previously reported with minor modifications (Douglas et al., 1995). In brief, the samples were thawed at room temperature and homogenized. This and all subsequent operations were performed under dim red illumination that bleaches the visual pigments minimally. Fifty microliters of n-dodecyl β-D-maltoside (200 mM; Sigma-Aldrich, St. Louis, MO) was added to every sample and the resulting mixture rotated for 2 h at room temperature, followed by 10 min centrifugation (23,000 g) at 4°C. The supernatant was removed and placed in a quartz cuvette in a Shimadzu UV-2101PC spectrophotometer (Shimadzu, Kyoto, Japan). After an initial scan of the unbleached extract from 300 nm to 700 nm, the sample was exposed to monochromatic light (502 nm) for 3 minutes, shown to be enough to completely bleach rhodopsin (Longbottom et al., 2009), and rescanned. All absorption spectra were zeroed at 700 nm. Difference spectra were constructed using the pre- and post-bleach curves and the maximum optical densities at ~ 500 nm determined, representing the amount of the extracted visual pigment.

### Example 6 - R9AP over-expression in rods and increased speed of photoreceptor deactivation

RGS9, Gβ5, and R9AP are obligate members of the regulatory GTPase complex. To study the effect of AAV-mediated R9AP over-expression on the GTPase complex in the rods, the level and distribution of RGS9 was examined following subretinal injection of rAAV2/8.Rho.mR9ap in *Cnga3-*/*-* mice. These mice have normal rod function but absent cone function and serve as a model of achromatopsia. Four weeks later, treated retina showed increased immunoreactivity against RGS9 throughout the photoreceptor layer in the treated compared to the untreated retinas (Figure 4A). Westernblot analysis further confirmed the increased RGS9 protein expression in the treated retina (Figure 4B). These results indicated that the over-expression of R9AP using AAV2/8 effectively increased the level of catalytic component RGS9 and the GTPase complex.

Next the functional effect of AAV2/8-mediated R9AP over-expression on rod phototransduction was studied by applying paired-flash ERG (Lyubarsky and Pugh 1996). In this paradigm, a pair of identical flash intensity is delivered with a variable inter-stimulus interval and the recovery of the second response relative to the first is measured. In the rod photoreceptor pathway, the speed of the a-wave (originating from photoreceptors) recovery is dependent on the speed of the deactivation. It was found that the time constant (σ) for 50% recovery of a-wave amplitude was reduced by -60% in the *Cnga3-*/*-* eyes injected with rAAV2/8.CMV.mR9ap (σ = ~ 2.99 sec ) compared to the untreated eyes (σ = ~ 7.38 sec; Figure 4C). Similarly, an increased speed of a-wave recovery was observed using rhodopsin promoter (rAAV2/8.Rho.mR9ap; σ = ∼ 2.74 sec; Figure 4C) in the same mouse line (*Cnga3-*/*-*) or the same virus (rAAV2/8.CMV.mR9ap) in another cone-defective mouse line (Pde6c-/-; Figure 8).

These observations indicated that the subretinal injection of rRAAV2/8.CMV.mR9ap or rAAV2/8.Rho.mR9ap can significantly increase the deactivation speed of the rod phototransduction through increasing the level of RGS9 and GTPase complex.

### Example 7 - "Photopic shift" of rod function by over-expression of R9AP

To investigate if an increased deactivation speed achieved by overexpression of R9AP and GTPase complex in the rods could alter the operating range of the photoreceptor function, dark-adapted 6 Hz flicker ERGs were recorded using incremental flash intensities. The eyes treated with rAAV2/8.CMV.mR9ap or rAAV2/8.Rho.mR9ap showed increased responses to brighter flashes compared to the untreated eyes. This resulted in an elevation the upper threshold of the response by up to ~2 log units (Figure SA), with little effect on the maximal photoresponse (151 ± 17 µV in the treated vs 162 ± 29 µV in the untreated eyes; average ± standard error of the mean ). As expected, this "photopic shift" in the operating range of the rods was accompanied by a reciprocal elevation the lower threshold of the response by up to ~1.5 log units. Meanwhile, upper threshold of ERG responses of wild-type eyes and *Gnat1-*/*-* eyes, both with functional cones, were elevated by ~4.0 log units compared to that of the untreated *Cnga3-*/*-* eyes. Similar results were obtained when rAAV2/8.Rho.mR9ap was injected into *Pde6c-*/*-* mice (Figure 9). As a consequence, the treatment allowed the rods to respond to flashes of longer durations (Figure 5B) and to flashes under a cone-isolating background illumination (Figure 5C). These include conditions where the untreated rods showed virtually no response.

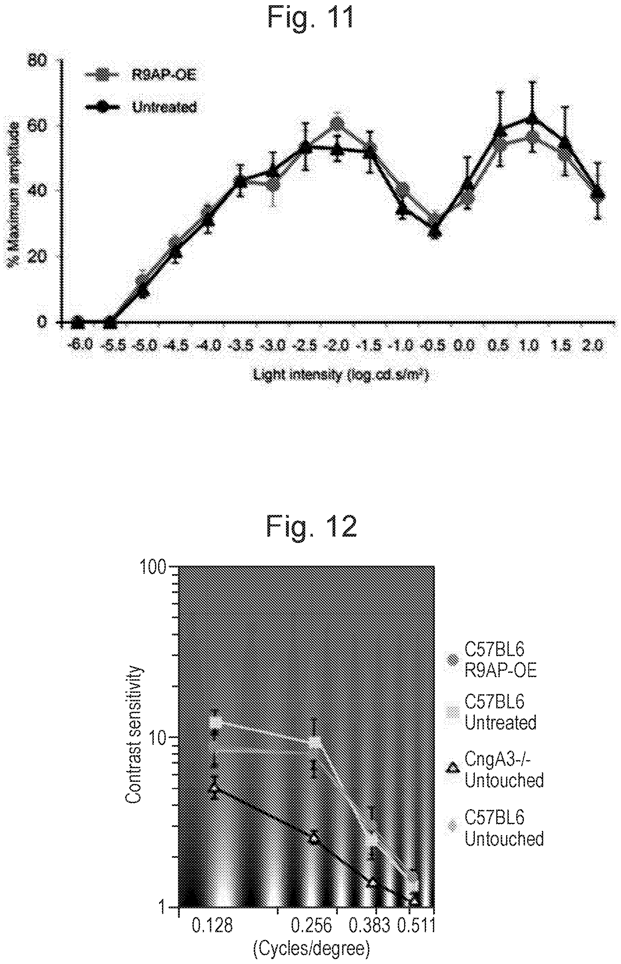

Taken together, these results established that R9AP-over expression in rods results in their desensitization and endows the cells to gain photopic function in exchange for scotopic function. This therapeutic induction of "photopic shift" of the rod function lasted at least for 5 months without overt evidence of retinal degeneration (Figure 10). Meanwhile, the treatment of wildtype mice using the same viral vectors failed to show a measurable change in retinal function (Figure 11).

### Example 8 - Rod bipolar pathway accommodates the transmission of altered rod function.

This work has established that an overexpression of R9AP in rods results in faster photoreceptor deactivation kinetics and allows the neuron to respond to larger amount of photons. Meanwhile, the accelerated deactivation should also result in a shorter duration of the neurotransmitter release at the photoreceptor synaptic terminal. Therefore, it was assessed whether the down-stream rod bipolar signaling is affected by the treatment. First the speed and the extent of transmission of signals from the photoreceptors to the bipolar cells to a short single flash was studied by measuring the implicit time and amplitudes of the a-wave (originating from the photoreceptors) and b-wave (originating from the bipolar cells) using ERG (Figure 6a). Overall, slightly smaller but a nearly identical intensity-response curve was observed for the treated and the untreated eyes for both a-wave and b-wave. The small difference observed may reflect either the true consequence of accelerated photoreceptor deactivation or merely the neural damage induced by the subretinal injection. The a-wave implicit time marks the point at which the bipolar cell-driven b-wave becomes detectable. We also observed a small delays in the a-wave and the b-wave implicit times, indicating a modest delay exists in the transmission of neural signals from photoreceptors to bipolar cells. Nevertheless, a relatively large variation of ERG responses between individuals indicate a small delay or reduction in rod response do not necessarily translate into visual dysfunction (Birch and Anderson 1992).

Therefore, these results indicated that, in principle, the bipolar cells almost fully accommodate the alteration of photoreceptor function and, importantly, display an appropriate dose-response relationship.

### Example 9 - R9ap over-expression results in improved Contrast Sensitivity Function

Next, it was asked if the "photopic shift" of the rod function by R9AP over-expression were consequently translated into improved visual performance under light by measuring optokinetic response to rotating sinusoidal gratings under the brightest recording condition possible with a standard computer monitor (62 cd/m²) (Carvalho et al., 2011). The unique advantage of this behavioral test is that visual function of each eye can be studied separately; the function of the right eye can be probed by responses to counterclockwise (CCW) gratings and the left eye by clockwise (CW) stimuli (Douglas et al., 2005). The spatial contrast sensitivity function (CSF) was studied with a fixed temporal frequency of 6.0 Hz and found that *Cnga3-*/*-* mice had reduced CSF compared to the wildtype mice (Figure 7). CSF, a function of contrast sensitivity and visual acuity, displayed the estimate range of visual perception (animals could presumably perceive the gratings under the curve but not above; Figure 7A). Intriguingly, an 8.0-fold (P = 0.005) and 5.4-fold (P = 0.011) increase in the sensitivity using gratings of both 0.128 and 0.256 cycles/degree (c/d), respectively, was observed when contrast sensitivity of the treated and the untreated eyes were compared (Figure 7A left panel). No clear alteration of contrast sensitivity was observed for gratings of 0.383 (P = 0.056) and 0.511 (P = 0.111) c/d. Interestingly, the average sensitivity of the treated eye in *Cnga3-*/*-* mice exceeded that of the wild-type controls with normal cone function. However, when wild-type mice were treated with the same viral construct, CSFs were not different between the treated and the untreated eyes (Figure 12).

Having established that R9AP overexpression results in gain of visual performance when viewing maximally bright monitor settings, it was sought to determine if this gain-of-vision is sustainable. This is of a valid concern considering that the regeneration of visual pigment in rods is known to be considerably slower than that of the cones (Wang and Kefalov 2011). First, it was assessed if the treatment results in alteration in the speed of visual pigment bleaching. It was found that an exposure of the treated and untreated eyes to a bright light for 5 minutes did not yield any difference in the levels of residual bleachable visual pigment (Figure 7B lower left panel). Second, the amount of bleachable rhodopsin in the eye was studied after exposing the *Cnga3-*/*-* mice for a variable amount of time to the same experimental condition carried out for CSF measurement. The results showed that visual pigment level remained stable without evidence of reduction throughout 2 hours' exposure to the visual stimuli similarly for the treated and the untreated eyes (Figure 7B lower right panel). These results indicated that the gain of visual perception in the treated *Cnga3-*/*-* mice is supported by sufficient supply of rhodopsin molecules and is sustainable.

### REFERENCES

Curcio, C.A., et al. The Journal of comparative neurology 292, 497-523 (1990). Dryja, T.P. American journal of ophthalmology 130, 547-563 (2000).
Hess, R.F. & Nordby, K. The Journal of physiology 371, 365-385 (1986). Nishiguchi, K.M., et al. Human mutation 25, 248-258 (2005).
Kohl, S., et al. Nature genetics 19, 257-259 (1998).
Chang, B. et al. P.N.A.S 106, 19581-19586 (2009).
Thiadens, A.A., et al. American journal of human genetics 85, 240-247 (2009). Burns, M.E. & Pugh, E.N. Biophys J 97, 1538-1547 (2009).
Burns, M.E. & Pugh, E.N. Physiology 25, 72-84 (2010).
Baseler, H.A., et al. Nature neuroscience 5, 364-370 (2002).
Martemyanov, K.A. & Arshavsky, V.Y. Prog MolBiol Transl 86, 205-227 (2009). Krispel, C.M., et al. Neuron 51, 409-416 (2006).
Cowan, C.W., et al. P.N.A.S. 95, 5351-5356 (1998).
Zhang, X., Wensel, T.G. & Kraft, T.W. The Journal of neuroscience: the official journal of the Society for Neuroscience 23, 1287-1297 (2003).
Nishiguchi, K.M., et al. Nature 427, 75-78 (2004).
Michaelides, M., et al. Ophthalmology 117, 120-127 e121 (2010).
Han, X. et al. Front Syst Neurosci. 5, 18 (2011).
Busskamp, V., et al. Science 329, 413-417 (2010).
Fu, Y., Yau, K.W. Pflugers Arch. 454, 805-819 (2007).
Pugh, E.N. Jr et al. Curr Opin Neurobiol. 9, 410-418 (1999).
de Jong, P.V.T.M. N EnglJMed 355, 1474-1485 (2006).
Wässle, H. NatRev Neurosci. 5, 747-757 (2004).
Soucy, E. et al. Neuron 21, 481-493 (1998).
Hack, I. et al. P.N.A.S. 96, 14130-14135 (1999).
Li, W. et al. Nat Neurosci 13, 414-416 (2010).
Biel, M. et al. P.N.A.S. 96, 7553-7557 (1999).
Umino, Y. et al. JNeurosci 28, 189-198 (2008).
Cangiano, L. et al. J Physiol. 590, 3841-3855 (2012).
Bemdt, A. et al. Science 344, 420-424 (2014).
Wietek, J. et al. Science 344, 409-412 (2014).
Calvert, P.D. et al. P.N.A.S 97, 13913-13918 (2000).
Gao, G.P. et al. P.N.A.S 99, 11854-11859 (2002).
Lyubarsky, A.L., et al. Vision research 44, 3235-3251 (2004).
Weymouth, A.E. & Vingrys, A.J. Progress in retinal and eye research 27, 1-44 (2008). Seeliger, M.W., et al. Nature genetics 29, 70-74 (2001).
Douglas, R.M., et al. Visual neuroscience 22, 677-684 (2005).
Douglas, R.H., et al. Journal of Comparative Physiology a-Sensory Neural and Behavioral Physiology 177, 111-122 (1995).
Longbottom, R., et al. P.N.A.S. 106, 18728-18733 (2009).
Lyubarsky, A.L. & Pugh, E.N., Jr. The Journal of neuroscience : the official journal of the Society for Neuroscience 16, 563-571 (1996).
Birch, D.G. & Anderson, J.L. Archives of ophthalmology 110, 1571-1576 (1992). Carvalho, L.S., et al. Human molecular genetics 20, 3161-3175 (2011).
Wang, J.S. & Kefalov, V.J. Progress in retinal and eye research 30, 115-128 (2011). Taylor, A. W., Ocular immune privilege, Eye, 23, 1885-1889 (2009).
Natkunarajah, M. et al., Assessment of ocular transduction using single-stranded and self-complementary recombinant adeno-associated virus serotype 2/8, Gene Ther. 15, 463-467 (2008).
Choi, V. W. et al., AAV hybrid serotypes: improved vectors for gene delivery, Curr. Gene Ther., 5, 299-310 (2005).
Wu, Z. et al., Adeno-associated virus serotypes: vector toolkit for human gene therapy, Mol. Ther., 14, 316-327 (2006).
Chuong, A.S. et al., Non-invasive optical inhibition with a red-shifted microbial rhodopsin, Nat Neurosci., 17, 1123-1129 (2014).

## Claims

1. An adeno-associated virus (AAV) vector comprising a nucleic acid encoding a gene product that is light-sensitive and/or that modulates endogenous light-sensitive signalling in a photoreceptor cell, for use in a method of improving vision in a patient with cone photoreceptor dysfunction and/or degeneration by introduction of said nucleic acid into healthy rod photoreceptors in the retina of the patient and expression of said gene product therein, such that the range of light intensities to which the rod photoreceptor responds is extended and/or the speed at which the rod photoreceptor responds to light is increased, wherein:
the gene product is ArchT, Jaws (cruxhalorhodopsin), iC1C2, or R9AP;
the capsid of the AAV vector is an AAV8 capsid, and
the nucleic acid is expressed under the control of a rod-specific promoter.

2. An AAV vector for use according to claim 1, wherein the genome of the AAV vector is derived from AAV2.

3. An AAV vector for use according to claim 1 or claim 2, wherein the patient suffers from macular degeneration, achromatopsia or Leber congenital amaurosis.

4. An AAV vector for use according to claim 3, wherein the macular degeneration is age-related macular degeneration (AMD), an inherited macular degeneration condition or an inherited cone dystrophy.

5. A vector for use according to claim 4, wherein the age-related macular degeneration (AMD) is wet or neovascular AMD or geographic atrophy.

6. An AAV vector for use according to any of the preceding claims, wherein rod photoreceptor signalling is extended into the mesopic and/or photopic illumination range.

7. An AAV vector for use according to any of the preceding claims, wherein the rod photoreceptors exhibit improved modulation strength and/or faster activation/inactivation kinetics.

8. An AAV vector for use according to any of the preceding claims, wherein the mesopic and/or photopic vision of the patient is improved.

9. An AAV vector for use according to any of the preceding claims, wherein the rod-specific promoter is a Rhodopsin (Rho), Neural retina-specific leucine zipper protein (NRL) or Phosphodiesterase 6B (PDE6B) promoter.

10. The AAV vector for use according to any one of claims 1 to 9 wherein the use is in combination with another therapy for the treatment or prevention of vision disorders.

11. An AAV vector for use according to any of the preceding claims, wherein the gene product is ArchT or R9AP.

## Patentansprüche

1. Adeno-assoziierter Virus- (AAV) Vektor, der eine Nukleinsäure umfasst, die für ein Genprodukt codiert, das lichtempfindlich ist und/oder die endogene lichtempfindliche Signalgebung in einer Photorezeptorzelle moduliert, zur Verwendung in einem Verfahren zur Verbesserung des Sehvermögens bei einem Patienten mit Zapfenphotorezeptordysfunktion und/oder -degeneration durch Einbringen der Nukleinsäure in gesunde Stäbchenphotorezeptoren in der Netzhaut des Patienten und Expression des Genprodukts darin, so dass der Bereich der Lichtintensitäten, auf den der Stäbchenphotorezeptor reagiert, erweitert wird und/oder die Geschwindigkeit, mit der der Stäbchenphotorezeptor auf Licht reagiert, erhöht wird, wobei:
das Genprodukt ArchT, Jaws (Cruxhalorhodopsin), iC1C2, oder R9AP ist;
das Kapsid des AAV-Vektors ein AAV8-Kapsid ist; und
die Nukleinsäure unter der Steuerung eines stabspezifischen Promotors exprimiert wird.

2. AAV-Vektor zur Verwendung nach Anspruch 1, wobei das Genom des AAV-Vektors von AAV2 abgeleitet ist.

3. AAV-Vektor zur Verwendung nach Anspruch 1 oder Anspruch 2, wobei der Patient an Makuladegeneration, Achromatopsie oder Leberscher kongenitaler Amaurose leidet.

4. AAV-Vektor zur Verwendung nach Anspruch 3, wobei die Makuladegeneration eine altersbedingte Makuladegeneration (AMD), eine vererbte Makuladegeneration oder eine vererbte Zapfendystrophie ist.

5. Vektor zur Verwendung nach Anspruch 4, wobei es sich bei der altersbedingten Makuladegeneration (AMD) um feuchte oder neovaskuläre AMD oder geographische Atrophie handelt.

6. AAV-Vektor zur Verwendung nach einem der vorhergehenden Ansprüche, wobei die Stäbchenphotorezeptor-Signalisierung in den mesopischen und/oder photopischen Beleuchtungsbereich ausgedehnt wird.

7. AAV-Vektor zur Verwendung nach einem der vorangehenden Ansprüche, wobei die Stäbchenphotorezeptoren eine verbesserte Modulationsstärke und/oder eine schnellere Aktivierungs-/Inaktivierungskinetik aufweisen.

8. AAV-Vektor zur Verwendung nach einem der vorangehenden Ansprüche, wobei das mesopische und/oder photopische Sehen des Patienten verbessert wird.

9. AAV-Vektor zur Verwendung nach einem der vorhergehenden Ansprüche, wobei der stäbchenspezifische Promotor ein Rhodopsin- (Rho), neuraler retinaspezifischer Leuzin-Zipper-Protein- (NRL) oder Phosphodiesterase 6B- (PDE6B) Promotor ist.

10. AAV-Vektor zur Verwendung nach einem der Ansprüche 1 bis 9, wobei die Verwendung in Kombination mit einer anderen Therapie zur Behandlung oder Prävention von Sehstörungen erfolgt.

11. AAV-Vektor zur Verwendung nach einem der vorhergehenden Ansprüche, wobei das Genprodukt ArchT oder R9AP ist.

## Revendications

1. Vecteur de virus adéno-associé (AAV) comprenant un acide nucléique codant pour un produit génique qui est sensible à la lumière et/ou qui module la signalisation endogène sensible à la lumière dans une cellule photoréceptrice, pour une utilisation dans un procédé d'amélioration de la vision chez un patient avec un dysfonctionnement et/ou une dégénérescence des photorécepteurs de cônes par introduction dudit acide nucléique dans des photorécepteurs de bâtonnets sains dans la rétine du patient et l'expression dudit produit génique dans ceux-ci, de telle sorte que la plage d'intensités lumineuses auxquelles le photorécepteur de bâtonnets répond soit étendue et/ou la vitesse à laquelle le photorécepteur de bâtonnets répond à la lumière est augmentée, dans lequel :
le produit génique est ArchT, Jaws (cruxhalorhodopsine), iC1C2 ou R9AP ;
la capside du vecteur AAV est une capside AAV8 ; et
l'acide nucléique est exprimé sous le contrôle d'un promoteur spécifique de bâtonnet.

2. Vecteur AAV pour une utilisation selon la revendication 1, dans lequel le génome du vecteur AAV est dérivé d'AAV2.

3. Vecteur AAV pour une utilisation selon la revendication 1 ou la revendication 2, dans lequel le patient souffre de dégénérescence maculaire, d'achromatopsie ou d'amaurose congénitale de Leber.

4. Vecteur AAV pour une utilisation selon la revendication 3, dans lequel la dégénérescence maculaire est une dégénérescence maculaire liée à l'âge (DMLA), un état de dégénérescence maculaire héréditaire ou une dystrophie conique héréditaire.

5. Vecteur pour une utilisation selon la revendication 4, dans lequel la dégénérescence maculaire liée à l'âge (DMLA) est une DMLA humide ou néovasculaire ou une atrophie géographique.

6. Vecteur AAV pour une utilisation selon l'une quelconque des revendications précédentes, dans lequel la signalisation du photorécepteur de cône est étendue dans la plage d'illumination mésopique et/ou photopique.

7. Vecteur AAV pour une utilisation selon l'une quelconque des revendications précédentes, dans lequel les photorécepteurs de bâtonnets présentent une force de modulation améliorée et/ou une cinétique d'activation/inactivation plus rapide.

8. Vecteur AAV pour une utilisation selon l'une quelconque des revendications précédentes, dans lequel la vision mésopique et/ou photopique du patient est améliorée.

9. Vecteur AAV pour une utilisation selon l'une quelconque des revendications précédentes, dans lequel le promoteur spécifique de la tige est un promoteur de la rhodopsine (Rho), de la protéine zipper leucine spécifique de la rétine neurale (NRL) ou de la phosphodiesterase 6B (PDE6B).

10. Vecteur AAV pour une utilisation selon l'une quelconque des revendications 1 à 9, dans lequel l'utilisation est en combinaison avec une autre thérapie pour le traitement ou la prévention des troubles de la vision.

11. Vecteur AAV pour une utilisation selon l'une quelconque des revendications précédentes, dans lequel le produit génique est ArchT ou R9AP.
